# EUROPEAN PATENT SPECIFICATION

(11) **EP 2 975 401 B1**
(45) Date of publication and mention of the grant of the patent: **25.12.2019**
(21) Application number: 14177705.2
(22) Date of filing: 18.07.2014
(51) Int. Cl.: G01N 33/50

(54) **IMPROVED METHOD OF MAPPING GLYCANS OF GLYCOPROTEINS IN SERUM SAMPLES**
VERBESSERTES VERFAHREN ZUR KARTIERUNG VON GLYCOPROTEINEN IN SERUMSPROBEN
PROCÉDÉ AMÉLIORÉ DE MISE EN CORRESPONDANCE DE GLYCANES DE GLYCOPROTÉINES DANS DES ÉCHANTILLONS DE SÉRUM

(43) Date of publication of application: 20.01.2016
(73) Proprietor: Hexal AG, 83607 Holzkirchen (DE)
(72) Inventor: Higel, Fabian, 82041 Oberhaching (DE); Seidl, Andreas, 82041 Oberhaching (DE); Demelbauer, Uwe, 82041 Oberhaching (DE)
(74) Representative: Dörries, Hans Ulrich

(56) References cited:
- WO-A1-2009/027041
- WO-A2-2005/111627
- SHUANG YANG ET AL: "Glycomic Analysis by Glycoprotein Immobilization for Glycan Extraction and Liquid Chromatography on Microfluidic Chip", ANALYTICAL CHEMISTRY, vol. 85, no. 21, 5 November 2013 (2013-11-05), pages 10117-10125, XP55148101, ISSN: 0003-2700, DOI: 10.1021/ac4013013
- JUSTIN M. PRIEN ET AL: "Mass Spectrometric-Based Stable Isotopic 2-Aminobenzoic Acid Glycan Mapping for Rapid Glycan Screening of Biotherapeutics", ANALYTICAL CHEMISTRY, vol. 82, no. 4, 15 February 2010 (2010-02-15), pages 1498-1508, XP55148093, ISSN: 0003-2700, DOI: 10.1021/ac902617t
- J. M. PRIEN ET AL: "A multi-method approach toward de novo glycan characterization: A Man-5 case study", GLYCOBIOLOGY, vol. 20, no. 5, 27 January 2010 (2010-01-27), pages 629-647, XP55148097, ISSN: 0959-6658, DOI: 10.1093/glycob/cwq012

## Description

### Field of the Invention

The present invention relates to a method for analyzing glycans of a recombinant glycoprotein in a liquid sample of a mammal. Specifically the method comprises a step of affinity purifying the recombinant glycoprotein from the sample, enzymatically releasing a glycan containing fragment from the immobilized glycoprotein, adding a reference standard containing isotopically labeled glycans, fluorescently label the glycans and analyzing the glycans using LC-MS. The present invention also relates to a method further comprising analyzing the glycans of the immobilized recombinant glycoprotein fragment, further comprising a pre-clearing step of the liquid sample, and releasing the glycans from the immobilized recombinant glycoprotein fragment. This method is particularly suited to measure pharmacokinetic parameters of a recombinant glycoprotein, such as a biopharmaceutical, in a mammal in clinical or pre-clinical studies. The present invention may be used with liquid chromatography-mass spectrometry, preferably reverse phase nano-liquid chromatography-mass spectrometry (RP-nano-LC-MS) technology. This allows for the use of a small sample volume and the possibility to operate with high throughput, such as in a 96-well plate sample preparation. It also provides high sensitivity and allows analysis of glycan structures individually.

### Background of the Invention

Biological drugs belong to the most complex pharmaceuticals. One important class of biological drugs are glycoproteins, and in particular monoclonal antibodies (mAbs), which are typically glycoproteins with a molecular mass of about 150 kDa. In recent years the glycosylation of therapeutic proteins came into focus as several investigations showed that these structures may have an effect on the safety and efficacy of the corresponding biological drugs. Further, possible effects of glycan structures on the pharmacokinetics (PK) have become of interest.

IgGs have a conserved N-glycosylation site on each heavy chain at their Fc part, usually around amino acid 297. The heterogeneity of the N-glycans attached to these sites is very high and more than a dozen different glycans can be found. Identification and quantification of glycans from this mixture is sophisticated and demands a comprehensive analytical approach. Structural isomers of several glycans make the discrimination even more difficult.

N-glycosylation is a critical issue for the safety and efficacy of biopharmaceuticals and has a possible influence on the pharmacokinetic of a glycoprotein, such as for therapeutic antibody or therapeutic Fc-fusion proteins. Hence, comprehensive analysis of individual glycan structures of a glycoprotein on the pharmacokinetics is necessary. The interest in Fc glycans involves the following major topics, the role of Fc glycans in antibody-dependent cell-mediated cytotoxicity (ADCC) and complement dependent cytotoxicity (CDC) and the role of glycans on clearance rate from the circulation as a result of glycoreceptors such as the asialoglycoprotein receptor that binds terminal galactose residues or mannose receptors that bind terminal mannose and N-acetylglucosamine residues.

N-glycosylation can be studied after enzymatic release of the glycans by PNGaseF using liquid chromatography, mass spectrometry or by a combination of both technologies (Chen and Flynn, Analytical Biochemistry 2007, 370, 147-61). To increase sensitivity, the N-glycans are often derivatized with fluorescence labels. For quantitative analytics of N-glycans, labeling with a fluorescence dye through reductive amination is widely used, offering high sensitivity. Every N-glycan carries only one label independent of size or branching, therefore, quantitative information can be obtained from the fluorescence signal intensity. The tag can not only provide UV or fluorescence detection but also the efficiency in electrospray ionization can be improved. For the analysis of sialic acid-containing glycans, labeling with a negatively charged tag, like 2-aminobenzoic acid (2-AA) or 8-aminonaphthalene-1,3,6-trisulfonic acid (ANTS), and MS detection in negative ionization mode is frequently used (Prien et al., Analytical Chemistry 2010, 82, 1498-508).

Negatively charged labeling, in combination with negative ionization in MS, has been frequently used to analyze acidic N-glycans (Prien et al., Glycobiology 2010, 20, 629-47). For the characterization of oligomannose structures, 2-AA derivatized N-glycans were analyzed by a combination of reversed phase chromatography and negative mode mass spectrometry (Prien et al., Glycobiology 2010, 20, 629-47).

WO 2009/027041 A1 is concerned with a method for the production of a glycosylated heterologous polypeptide comprising *inter alia* the steps of obtaining a sample from a crude fermentation broth, incubation of the sample with magnetic affinity beads, releasing glycans from the immobilized glycosylated polypeptides and measuring a glycosylation profile. A publication by Yang et al. (Analytical Chemistry 2013, 85, 10117-10125) relates to a method for the high-throughput analysis of N-glycans from glycoproteins using glycoprotein immobilization for glycan extraction (GIG) coupled with liquid chromatography (LC) in an integrated microfluidic platform (chipLC).

In WO 2014/060551, an efficient and sensitive analysis of neutral and acidic N-glycans by RP-LC-MS in one approach is described. Previous methods have been mostly limited to the isomer separation of oligomannose type N-glycans. In WO 2014/060551 anthranilic acid (2-AA) is used to label N-glycans prior to separation using a reversed-phase liquid chromatography (RP-LC) column under acidic conditions using formic acid. Negatively charged 2-AA offers stronger retention on the reversed phase column than 2-aminobenzamide (2-AB) in RP-LC and allows efficient ionization and detection of 2-AA labeled N-glycans. The acidic conditions used for the RP-LC lead to an efficient separation of acidic 2-AA N-glycans carrying terminal sialylation without the need for an ion-pairing reagent. This method may be used with RP-nano-LC-MS and a 96-well plate sample preparation, which allows attomolar sensitivity and high throughput.

N-glycosylation analysis is sophisticated because of countless N-glycan variants which may be attached to the protein molecules and the huge differences in their relative amounts. For example, for recombinant human IgG antibodies relative amounts of individual oligomannose N-glycans may range from 0.02% to more than 70% for the most abundant N-glycan, differences that cover three orders of magnitude (Higel et al., Analytical and Bioanalytical Chemistry 2013, 405, 2481-93). Technologies frequently used for N-glycan analysis are CE, HPAEC-PAD, HPLC, MALDI and ESI-MS and various

combinations of these technologies (Mariño et al., Nature Chemical Biology 2010, 6, 713-23). LC-MS is an advantageous combination as LC is able to separate glycan mixtures; afterwards glycan variants can then individually be identified and quantified by on-line MS. For various analytical applications, however, conventional LC-MS may not be sufficiently sensitive, especially for cases where the sample amount is strongly limited. During early biopharmaceutical development (clone or pool selection) and during pre-clinical studies, only minute amounts of recombinant protein from micro titer plates are usually available for protein and glycan analysis. For studying the influence of glycan variants on the pharmacokinetics of glycosylated biopharmaceuticals, the recombinant glycoprotein of interest is further present in a sample, e.g., serum, in admixture with other proteins, most of which are also glycosylated. Further, the amount of the recombinant glycoprotein of interest declines over time. Thus, high sensitivity, specificity and low sample consumption is required. Typically there is a large sample number, hence the glycoprotein of interest must be isolated from the sample in a reproducible manner ideally in a high throughput format and the analysis of the individual samples within a series of samples must be comparable to each other. Moreover, the glycoprotein may have more than one N-glycosylation site, with differences in glycan structures at each glycosylation site.

In proteomics, limitations in sample volume have been circumvented by reducing the dimensions of the analytical system, for example, by the use of nano-LC-MS. Approaches for N-glycan analysis by the use of nano-LC-MS are rare in the literature. Three investigations have reported feasibility of nano-LC for glycan analysis. Wuhrer *et al.* have miniaturized HILIC-MS to nanoscale for oligosaccharide analysis (Wuhrer et al., Analytical Chemistry 2004, 76, 833-8). They have analyzed underivatized N-glycans with femtomolar sensitivity. Avoiding glycan derivatization shortens sample preparation but the benefit of improved MS detection coming with the label is lost (Wuhrer et al., Analytical Chemistry 2004, 76, 833-8; Ruhaak et al., Analytical and Bioanalytical Chemistry 2010, 397, 3457-81). Kalay *et al.* have used normal phase nanoscale HPLC-MS with on-line fluorescence to analyze 2-AB N-glycans (Analytical Biochemistry 2012, 423, 153-162). Their approach resulted in long and time consuming gradients to achieve a good chromatographic resolution. One work utilizing nano-reversed phase chromatography (nano-RPC) has been published recently. Ritamo et al. (Analytical and Bioanalytical Chemistry 2013, 405, 2469-80) have used a nano-LC system to separate permethylated N-glycans. They have achieved separation for various structural isomers on a nano-RP-column. Permethylation of N-glycans, however, is complex and toxic reagents are used and formation of side products is rather likely which makes routine use questionable. It has been reported previously that RP-LC with on-line MS is broadly applicable for analysis of differently reducing-end labeled N-glycans (Chen and Flynn, Analytical Biochemistry 2007, 370, 147-61; Prater et al., Analytical Biochemistry 2009, 385, 69-79). An overall sensitivity for a single 2-AA labeled N-glycan, e.g., about 400 attomole using RP-nano-LC-MS was reported in WO 2014/060551. This method may further be combined with a 96 well based sample preparation work-flow.

Quantification of proteins by MS is frequently performed using stable heavy isotope peptides or proteins. Stable heavy isotope labeling of N-glycans in contrast is hardly possible due to the complex and multiple connected anabolic and catabolic carbohydrate pathways of eukaryotic cells. The controlled incorporation of isotopically labeled monosaccharides is far more challenging than the incorporation of isotopically labeled amino acids like lysine or arginine.

The role of glycans in clearing IgG molecules in circulation has been examined by various groups, including pre-clinical or clinical pharmacokinetic (PK) studies, in an attempt to determine the impact of Fc glycan changes on serum clearance. Two general approaches have been used in these studies. In one approach, antibodies have been prepared that are enriched in specific glycan forms, either through genetic mutation, addition of metabolic inhibitors, a combination of both, affinity purification or enzymatic treatment. These antibody samples are then injected into animals to determine the impact on the overall clearance rate. However, this approach has limitations because of the assumption that the only change in the molecule during enrichment is the glycan structure. Glycan heterogeneity can also lead to some ambiguity of the results. Also different variants of the same product need to be prepared and administered. Thus, this approach is not suitable for pre-clinical or clinical pK studies of biopharmaceuticals, such as therapeutic recombinant antibodies or fusion proteins.

In another approach, the glycan forms are analyzed after the drug has been administered and changes to the glycan pattern with circulation time are interpreted as differences in clearance rates. The post-administration collection approach can follow many more glycan forms simultaneously. Since changes to a single administered sample are followed, the impact of specific microheterogeneity can be monitored. To the best of our knowledge this approach has only been used in humans, but not in smaller mammals typically used in pre-clinical studies, where sample size is more limiting. However, analysis of glycans on pharmacokinetics as early as possible in the development of biopharmaceuticals is desirable, as well as reducing the sample size needed in clinical trials.

Alessandri et al (mAbs, 2012, 4(4), 509-520) analyzed a recombinant monoclonal IgG1 antibody from serum samples obtained from a human PK study. The antibody was purified from serum by affinity chromatography using its ligand cross-linked to sepharose beads. The glyans were released from the acidically eluted antibody, labeled with 2-aminobenzamide (2-AB) and analyzed by normal phase high performance liquid chromatography. However, the assay allowed analysis only up to 14 days post-administration in a rather high sample volume and is prone to contamination with other serum proteins.

Chen et al (Glycobiology 2009, 19(3), 240-249) analyzed monoclonal antibodies in serum samples of a pharmacokinetic study in humans. The antibodies were affinity purified using a ligand cross-linked to a resin and acidically eluted thereof. The released glycans were labeled with 2-aminobenzamide (2-AB) and analyzed by RP-HPLC/MS. This isolation method was capable of obtaining only 70% of the specific antibody from the serum. Also, this method is subject to interference from glycans released from other serum proteins or other glycans of the same molecule for glycoproteins with more than one glycosylation sites.

Goetze et al (Glycobiology 2011, 21(7), 949-959) analyzed monoclonal IgG1 and IgG2 antibodies in serum samples of a pharmacokinetic study in humans. The antibodies were affinity purified using the respective ligand cross-linked to a resin. The antibodies were acidically eluted and digested with either the endoproteinase Lys-C or trypsin. The glycosylated peptides were analyzed using LC-MS/MS. This method offers the advantage of being specific for the consensus Fc glycosylation of either human IgG1 or human IgG2. The applied peptide mapping approach reduces the potential pool of interfering impurities to only endogenous IgG of the same subclass as the mAb to be analyzed. However, this method requires a sample volume of 0.5 ml, which often may not be available, e.g., in preclinical studies using rodents. Also this analysis allows only for determining the relative proportion of N-glycan structures within a sample, but not for an independent analysis of single N-glycan structures.

None of these methods analyzed recombinant glycoproteins other than antibodies that may comprise more than one glycosylation site with structural differences at the glycosylation sites, such as in the two domains of a fusion protein. Further, there was a need for a method to analyze individual glycans of a recombinant glycoprotein in a series of mammalian samples, such as serum, rather than a proportion of the glycans within the sample. There was also a need for high sensitivity to allow for low sample consumption and a sample preparation to be operated in a high throughput manner.

### Summary of the Invention

Accordingly, the present invention relates to a method of analyzing glycans of a recombinant glycoprotein of interest in liquid samples of a mammal comprising a) immobilizing the recombinant glycoprotein of interest comprised in each of two or more liquid samples on a separate solid support coupled to an affinity ligand specific for the recombinant glycoprotein in the samples; b) releasing a glycan containing fragment of the recombinant glycoprotein of each of said samples from the solid support into separate eluates by enzymatic cleavage of the recombinant glycoprotein using an endoproteinase; c) optionally releasing the glycans from the glycan containing fragment of the recombinant glycoprotein of each of said samples in the separate eluates; d) labeling the glycans of each of said samples with a first stable isotope of a fluorescent label; and e) analyzing the labeled glycans of d) of each of said samples separately using LC-MS and comparing said two or more samples, wherein a reference standard comprising glycans labeled with a second stable isotope of the fluorescent label is added to each of said samples prior to step b), c), d), or e) and the reference standard is analyzed together with the labeled glycans of step e).

The present invention also relates to a method further comprising analyzing the glycans of a second glycan containing fragment of the recombinant glycoprotein of each of said samples that remains immobilized to the solid support in step b), wherein the recombinant glycoprotein is an Fc-fusion protein or an antibody, comprising the following steps: aa) pre-clearing each of the two or more liquid samples of a mammal to remove glycoproteins binding non-specifically to the solid support prior to step a), comprising aai) immobilizing Fc-containing glycoproteins on a solid support using an Fc-binding protein, wherein said Fc-binding protein is preferably selected from protein G or protein A. more preferably said Fc-binding protein is protein G; and aaii) eluting the Fc-containing glycoproteins; wherein the solid support used in said pre-clearing step is made of the same material as the solid support used in step a) of claim 1, but is not coupled to said affinity ligand; cc) releasing the glycans from the immobilized second glycan containing fragment of the recombinant glycoprotein of each of said samples remaining on the solid support following step b) into separate solutions; eedd) labeling the glycans of each of said samples with a first stable isotope of a fluorescent label; and ee) analyzing the labeled glycans of step dd) of each of said samples separately using LC-MS and comparing said two or more samples, wherein a reference standard comprising glycans labeled with a second table isotope of the fluorescent label is added to each of said samples prior to step cc), dd) or ee) and wherein the reference standard is analyzed together with the labeled glycans of step ee). The pre-clearing step aa) may comprise contacting the samples with a solid support, wherein the solid support is made of the same material as the solid support used in step a), but is not coupled to said affinity ligand.

According to the present invention the glycans to be analyzed may be N-glycans selected from the group consisting of high mannose type, hybrid type or complex type N-glycans.

The fluorescent label as used in the methods of the invention has a stable light and a stable heavy isotope variant and may be for example 2-amino benzamide (2-AB) or 2-amino benzoic acid (2-AA). Preferably, the fluorescent label is a ¹²[C] and ¹³[C] isotopic pair, more preferably a ¹²[C6] and ¹³[C6] isotopic pair, and even more preferably a ¹²[C₆]-2-aminobenzoic acid (¹²[C₆]-2-AA) and a ¹³[C₆]-2-aminobenzoic acid (¹³[C₆]-2-AA) isotopic pair.

The reference standard used in the methods according to the invention comprises glycans labeled with a stable (second) isotope of the fluorescent label, preferably with a stably heavy isotope of the fluorescent label. Preferably, the reference standard comprises at least the same glycan structures as analyzed in the sample. The reference standard allows for analyzing the glycan structures individually.

The recombinant glycoprotein analyzed in the methods of the invention is preferably a fusion protein or an antibody, more preferably an Fc-fusion protein or an antibody. In embodiments where the recombinant protein is an Fc-fusion protein or antibody the glycan containing fragment of the recombinant glycoprotein released from the solid support in step b) is preferably an Fc domain.

In one embodiment the recombinant glycoprotein is an antibody and the variable region of the antibody binds to the affinity ligand immobilized on the solid support, preferably the affinity ligand is an antigen. In a preferred embodiment the antibody is an IgG antibody, more preferably an IgG1 or IgG2 antibody and even more preferably a human or humanized IgG1 or IgG2 antibody.

In another embodiment the recombinant glycoprotein is an Fc-fusion protein comprising an Fc-domain and an effector domain and the effector domain binds to the affinity ligand immobilized on the solid support, wherein the affinity ligand is a binding partner or an antibody specifically binding to the effector domain of the Fc-fusion protein.

The enzyme used in the methods of the invention to release the glycan containing Fc-domain of the recombinant glycoprotein from the solid support is an endoproteinase, such as papain, ficin, cysteine protease SpeB (FabULOUS) or cysteine proteinase IdeS (FabRICATOR®), preferably the endoproteinase is the cysteine proteinase IdeS (FabRICATOR®).

In one embodiment of the methods of the invention, the recombinant protein is an antibody or an Fc-fusion protein and the pre-clearing step comprises aai) immobilizing the Fc-containing glycoproteins on a solid support using an Fc-binding protein, wherein said Fc-binding protein is preferably selected from protein G or protein A, more preferably said Fc-binding protein is protein G; and aaii) eluting the Fc-containing glycoproteins; wherein the solid support used in said pre-clearing step is made of the same material as the solid support used in step a), but is not coupled to said affinity ligand.

The LC-MS used in the methods of the present invention is preferably a reverse phase LC-MS or a NanoLC-MS, more preferably the LC-MS is a reverse phase NanoLC-MS.

In certain embodiments of the methods of the present invention, the solid support is a resin comprising microbeads, preferably sepharose beads, agarose beads or magnetic beads, more preferably sepharose beads. The affinity ligand may be coupled to the solid support via N-hydroxysuccinimide (NHS), cyanogen bromide, epoxy, carbodiimide or thiopropyl, preferably via N-hydroxysuccinimide (NHS).

In a preferred embodiment of the methods of the present invention, the two or more liquid samples of a mammal are prepared for analysis using a multi well filter plate, preferably in a 24, 96 or 384 well filter plate, more preferably in a 96 well filter plate.

The liquid samples of a mammal comprising the recombinant protein is a body fluid and is preferably selected from the group consisting of serum or plasma, urine, cerebral spinal fluid, amniotic fluid, saliva, sweat, ejaculate, tears, phlegm, vaginal secretion, vaginal wash and colonic wash; more preferably said samples are plasma or serum samples. Preferably the samples to be analyzed have a volume from about 1 µl to about 1000 µl, from about 5 µl to about 500 µl, from about 10 µl to about 200 µl, from about 10 µl to about 100 µl, from about 25 µl to about 100 µl, or from about 40 µl to about 75 µl, preferably of about 50 µl. In a preferred embodiment the two or more liquid samples of a mammal were obtained from the same subject. The mammalian liquid sample analyzed in the methods of the invention may be a human, a monkey, a rodent, a dog, a cat or a pig sample, preferably a rodent sample such as from mouse, rat, hamster or rabbit.

The methods of the present invention may be used to determine pharmacokinetic parameters of at least one specific glycan structure of the glycans of the recombinant glycoprotein of interest, preferably to determine the Cₘₐₓ, tₘₐₓ, AUC or t_{1/2} thereof.

In certain embodiment, the glycans are analyzed in an aliquot of each of said two or more liquid samples of a mammal and optionally the concentration of said recombinant glycoprotein is analyzed in a further aliquot of said two or more liquid samples of a mammal, e.g., by ELISA.

Preferably the aliquots to be analyzed have a volume from about 1 µl to about 1000 µl, from about 5 µl to about 500 µl from about 10 µl to about 200 µl, from about 10 µl to about 100 µl, from about 25 µl to about 100 µl, or from about 40 µl to about 75 µl preferably of about 50 µl

The present disclosure also relates to a method of preparing a glycoprotein based pharmaceutical composition comprising analyzing the glycans of a recombinant glycoprotein in liquid samples of a mammal according to the method of the invention and formulating the glycoprotein into said pharmaceutical composition.

In yet another aspect the invention relates to a method of analyzing glycans of an Fc-fusion protein containing an Fc-domain and an effector domain of interest in liquid samples of a mammal, comprising aa) pre-clearing each of the two or more liquid samples of a mammal comprising: immobilizing the Fc-fusion protein of interest comprised in each of said two or more liquid samples on a separate solid support using an Fc-binding protein, wherein said Fc-binding protein is preferably selected from protein G or protein A, more preferably said Fc-binding protein is protein G; and eluting the Fc-fusion protein; a) immobilizing the Fc-fusion protein of each of said samples on a separate solid support coupled to an affinity ligand specific for the effector domain of the Fc-fusion protein in the sample, wherein the affinity ligand is a binding partner or an antibody specifically binding to the effector domain of the Fc-fusion protein; b) releasing the Fc-domain of the Fc-fusion protein of each of said samples from the solid support into separate eluates by cleaving with an endopeptidase specific for Fc-fusion proteins; cc) releasing the glycans from the immobilized effector domain of the Fc-fusion protein of each of said samples remaining on the solid support following step b) into separate solutions; dd) labeling the glycans of each of said samples with a first stable isotope of a fluorescent label; and ee) analyzing the labeled glycans of step dd) of each of said samples separately using LC-MS and comparing said two or more samples; wherein a reference standard comprising glycans labeled with a second stable isotope of the fluorescent label is added to each of said samples prior to step cc) dd) or ee) and wherein the reference standard is analyzed together with the labeled glycans of step ee).

### Brief Description of the Figures

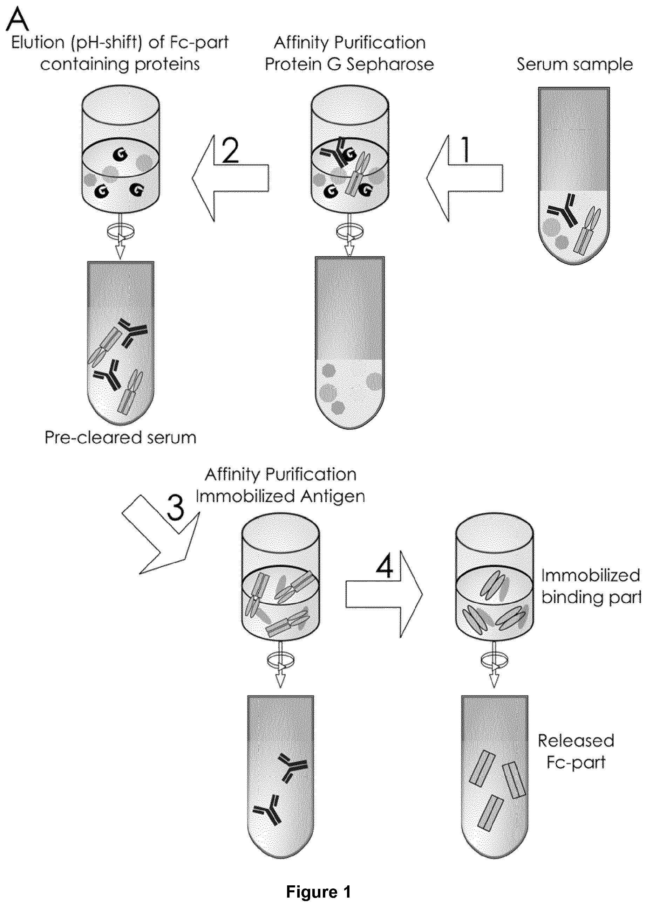

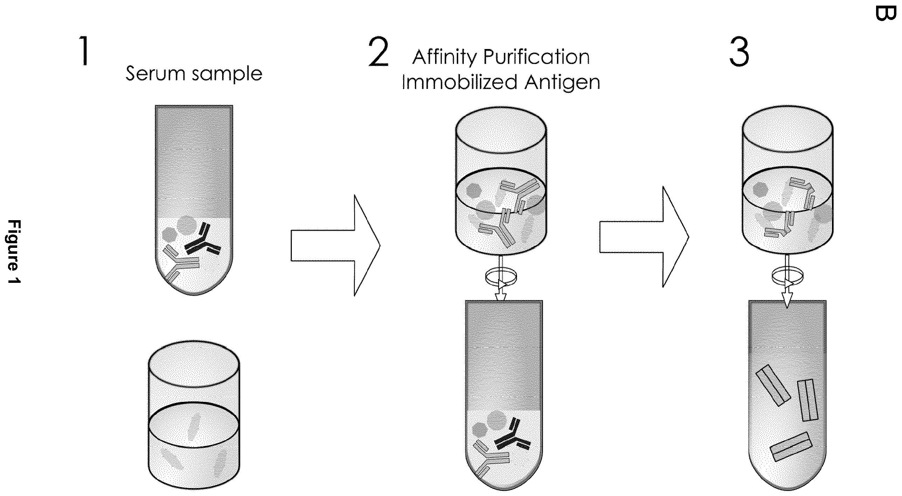

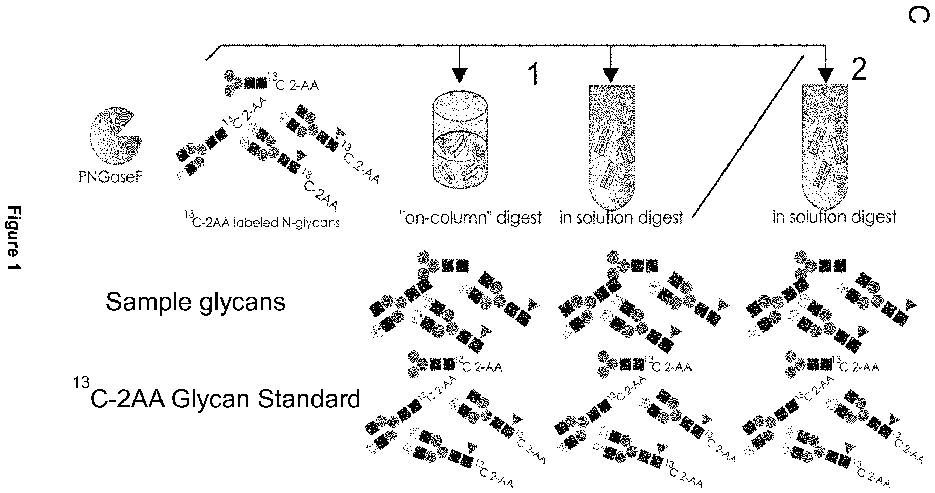

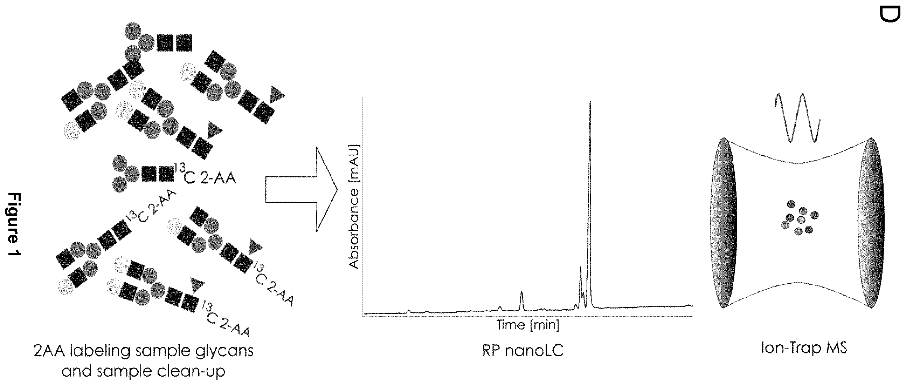

**Figure 1****:** Schematic illustration of the work flow of the glycan PK profiling method exemplified for Fc-fusion proteins and antibodies. The work flow shows A) an Fc-fusion protein specific sample preparation and B) an antibody specific sample preparation, C) N-glycan processing of the Fc-fusion protein sample (1) and the antibody sample (2) and D) glycan clean-up after labeling followed by RP NanoLC-MS analysis.

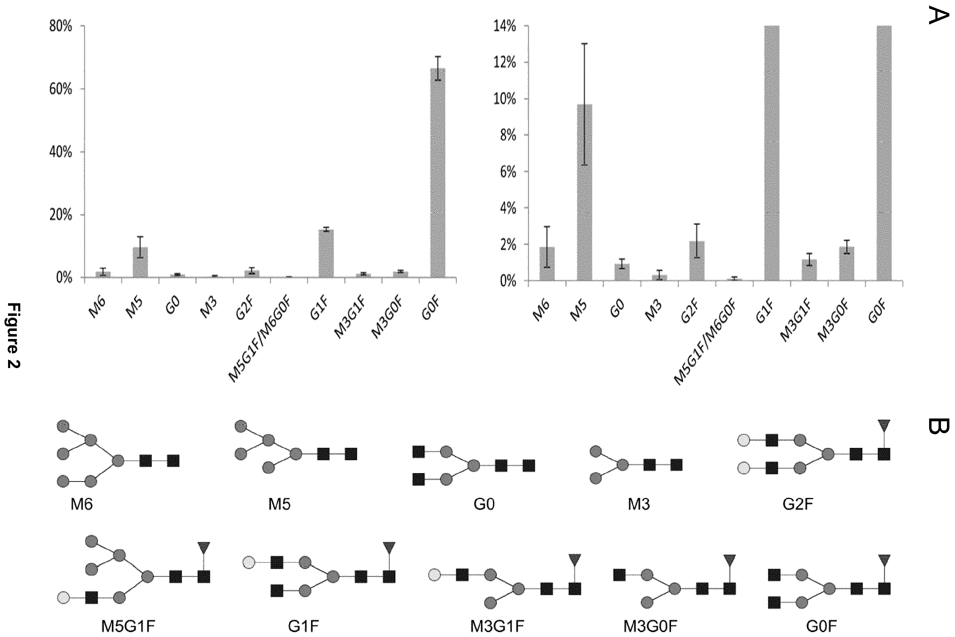

**Figure 2****:** A) Glycan map obtained from quality control (QC) samples showing the relative N-glycan composition (left) and a magnified view (right) showing the minor abundant N-glycans. The error bars represent the standard deviation. The two columns without error bars, G1F and G0F, extend beyond the graph in the magnified view. QC sample concentrations were between 10 µg/ml and 100 µg/ml. B) Schematic overview on the analyzed glycan structures, wherein GlcNAc = filled square, Fuc = triangle, Man = dark circles and Gal = light circle.

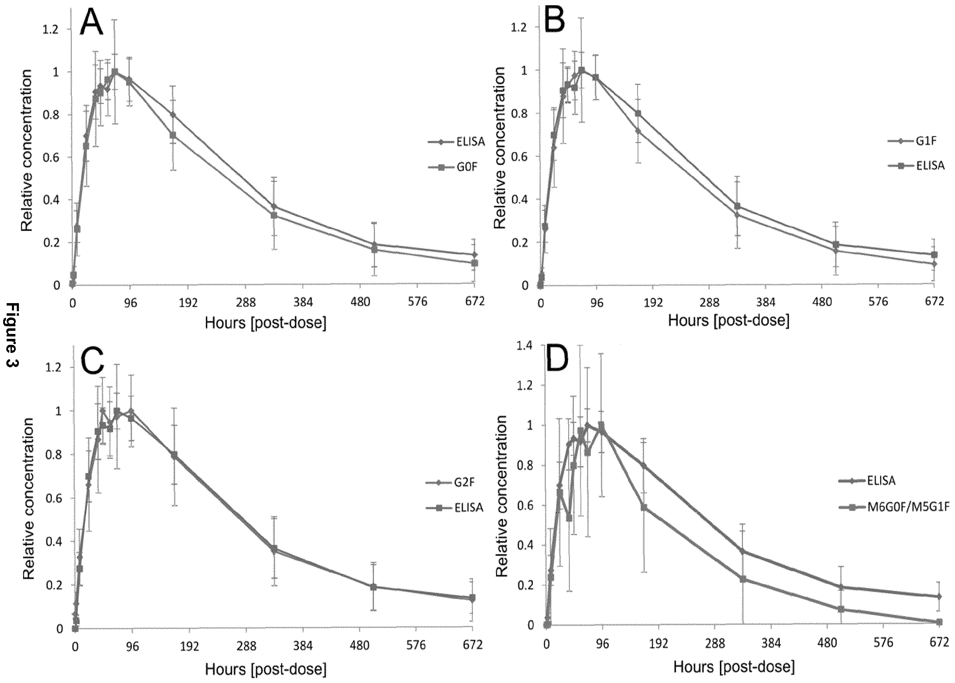

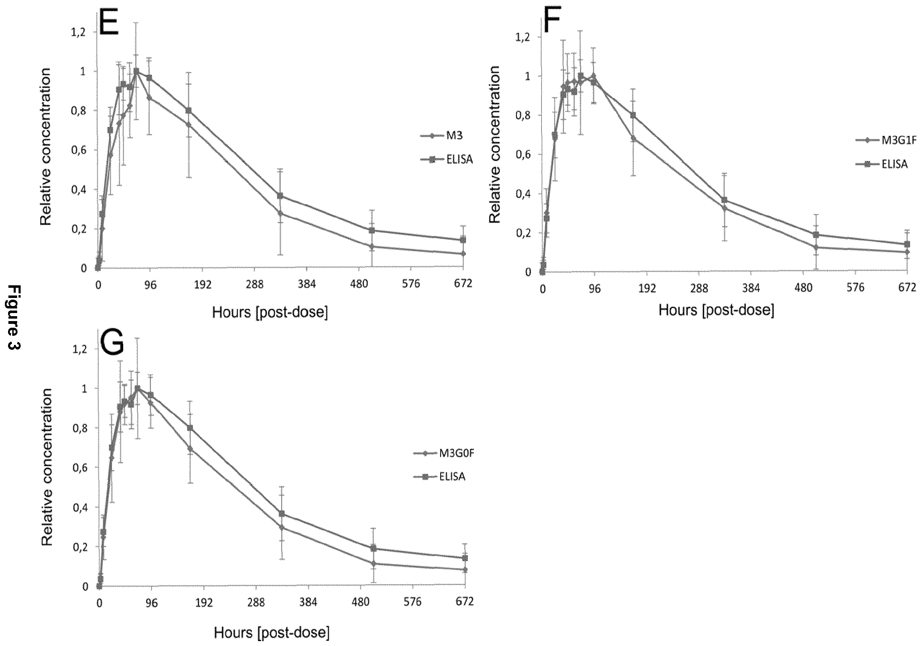

**Figure 3****:** Comparision of nanoLC-MS based mAb1 glycan PK data (filled diamonds) and ELISA data (filled squares). Independent results from four glycans relative to the maximum of each curve are shown to enable comparison (mean ± SD, n =15). In (A) PK profile of G0F (about 70% of glycans), in (B) PK profile of G1F, in (C) PK profile of G2F, in (D) PK profile of M6G0F/M5G1F (< 0.1% of glycans), in (E) PK profiles of M3, in (F) PK profile of M3G1F and in (G) PK profile of M3G0F are shown.

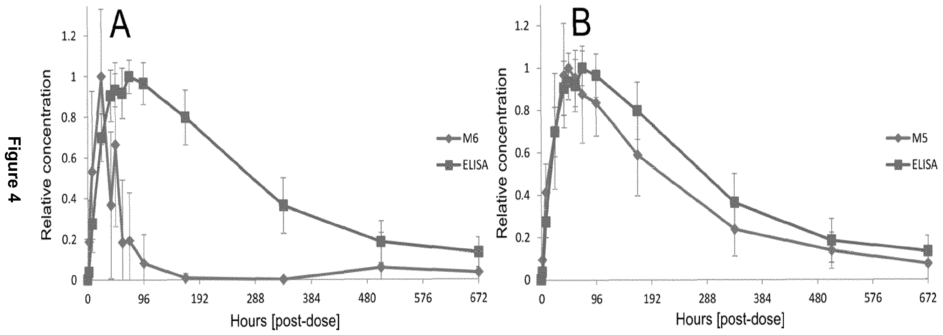

**Figure 4****:** Comparison of nanoLC-MS based mAb1 high mannose glycan PK profiles obtained by nanoLC-MS and ELISA profiles. (A) PK profile of M6 (filled diamonds) compared to ELISA (filled squares). (B) PK profile of M5 (filled diamonds) compared to ELISA (filled squares).

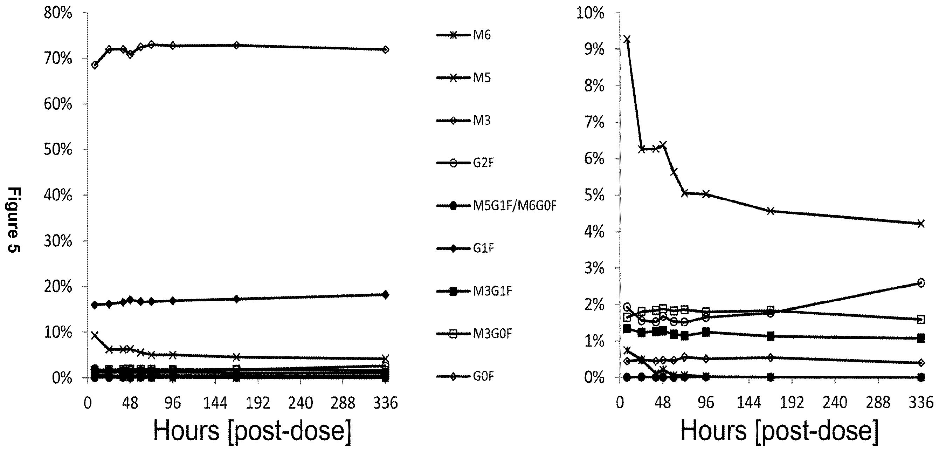

**Figure 5****:** Glycan Maps of mAb1 for each time point. Shown are mean percentage of recovered mAb1 N-glycans after single subcutaneous administration in rabbits. Full view (left) and magnified view (right) are shown.

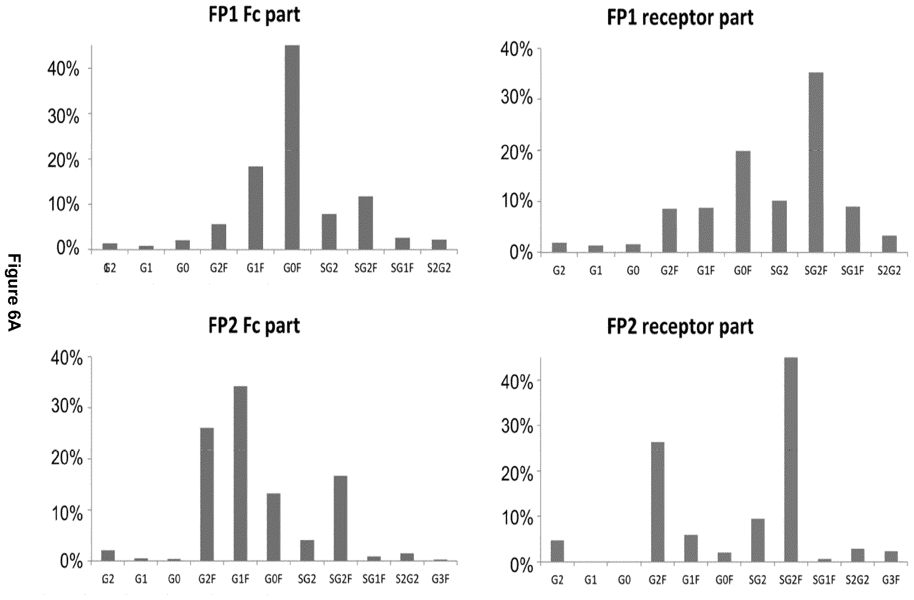

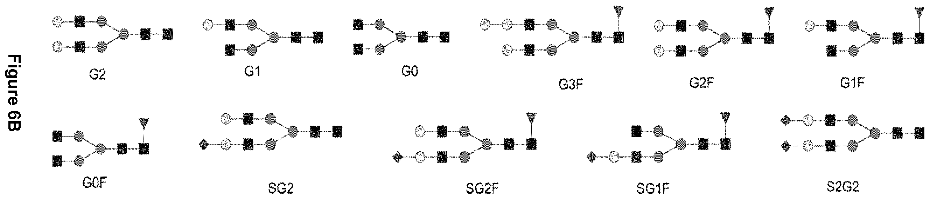

**Figure 6****:** Fusion protein N-glycans of the two different batches (FP1 and FP2) separated into Fc part and receptor part. A) Glycan maps show the relative N-glycan composition of FP1 Fc part (top left), FP1 receptor part (top right), FP2 Fc part (bottom left) and FP2 receptor part (bottom right). The magnified view shows the minor abundant N-glycans. B) Schematic overview on the analyzed glycan structures, wherein GlcNAc = filled square, Fuc = triangle, Man = dark circles, Gal = light circle and SA = diamond.

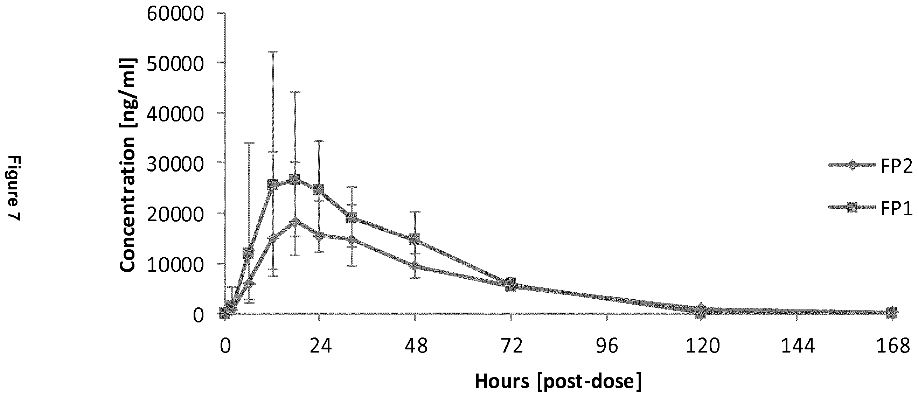

**Figure 7****:** PK profiles of FP1 and FP2 determined by ELISA. Error bars represent the variability between the animals (n=5).

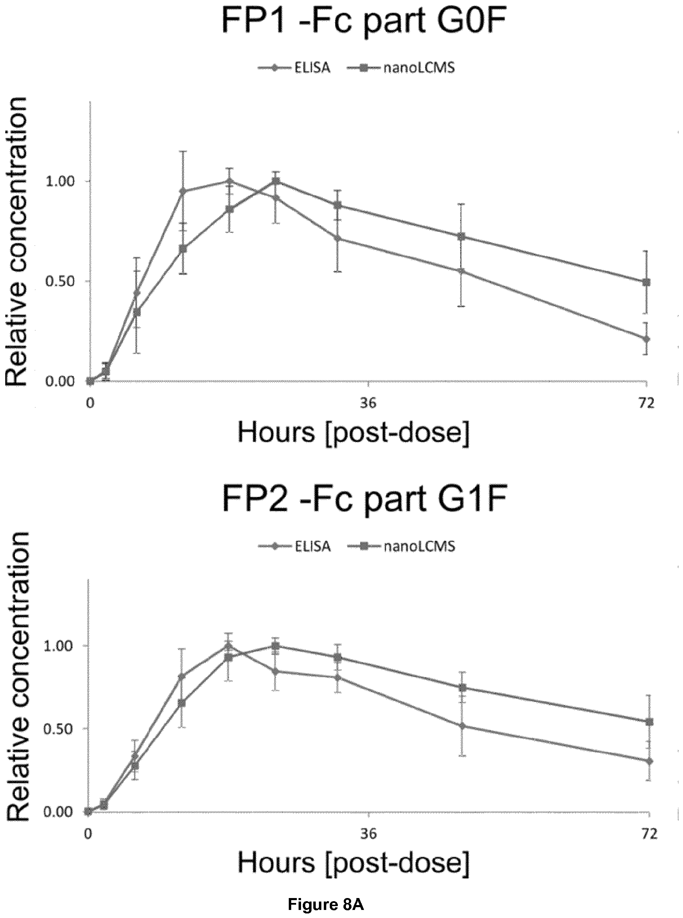

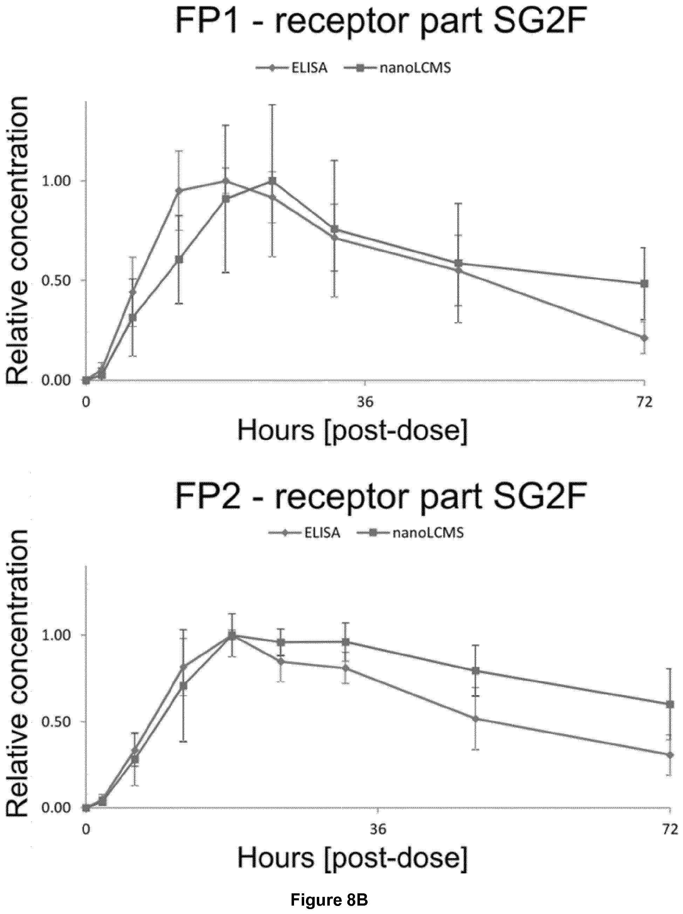

**Figure 8****:** Comparison of nanoLC-MS based fusion protein glycan PK profiles with ELISA profiles. Shown are the average PK profiles of the major N-glycans (A) of the Fc-domain of FP1 and FP2 as measured by NanoLC-MS (filled squares) and (B) of the receptor part (effector domain) of FP1 and FP2 as measured by NanoLC-MS (filled squares) compared to the relative protein concentration as measured by ELISA (filled diamonds). Provided is the mean with the standard deviation of 5 animals.

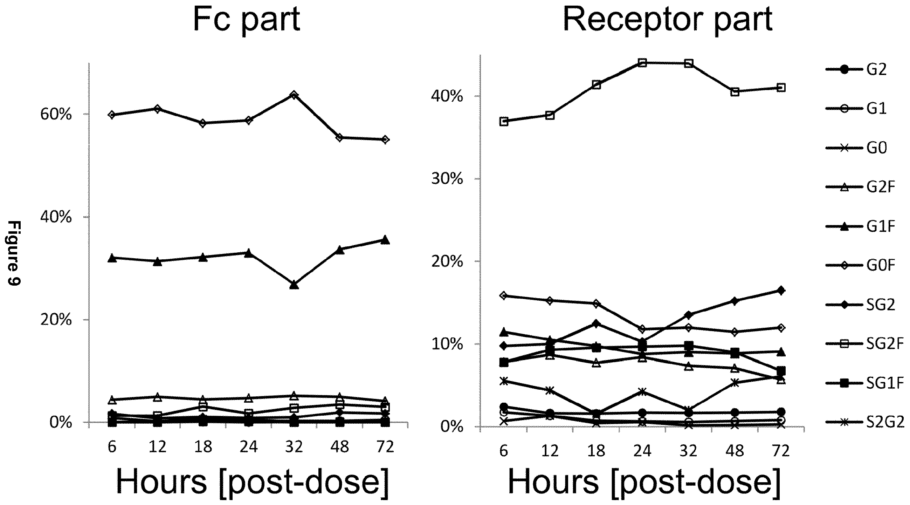

**Figure 9****:** Glycan Maps for N-glycans located on the FP1 Fc part (left) and the receptor part (right) are shown separately.

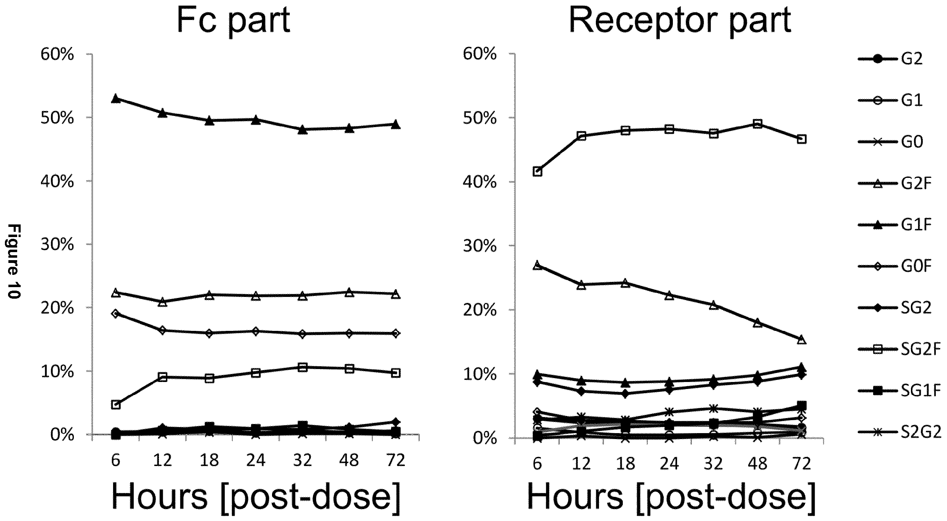

**Figure 10****:** Glycan Maps for N-glycans located on the FP2 Fc part (left) and the receptor part (right) are shown separately.

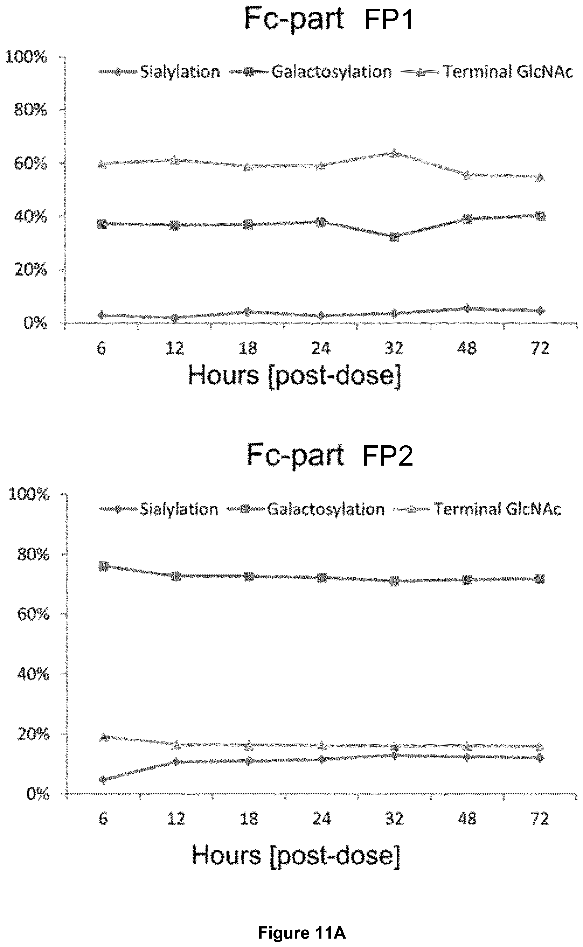

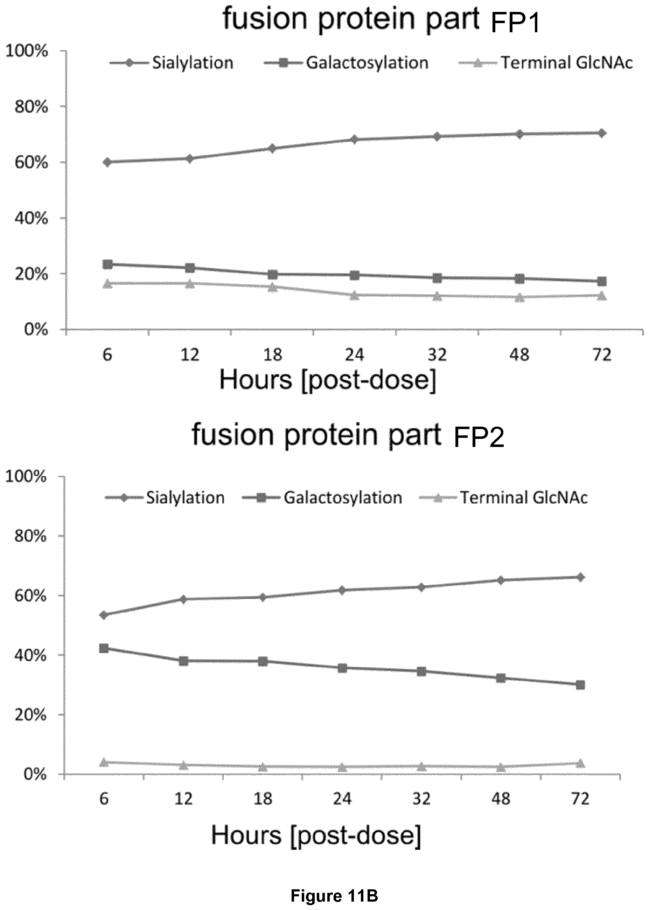

**Figure 11****:** Influence of terminal sugar moieties. Percentages of terminal sialylation (diamond), terminal galactosylation (squares) and terminal N-acetylglucosamine (triangle) are pictured (A) the for Fc-part of fusion protein 1 (FP1) and fusion protein 2 (FP2) and (B) for the receptor part (effector domain) of fusion protein 1 (FP1) and fusion protein 2 (FP2).

### Detailed Description of the Invention

As used herein, a "fluorescent label" is a fluorescent compound used in LC-MS analysis. Typically reductive amination is used to introduce a fluorescent label to oligosaccharides, facilitating detection in the subsequent separation, preferably such fluorescent label is 2-amino benzamide (2-AB) or 2-amino benzoic acid (2-AA). *2-AA* is 2-amino benzoic acid, also known as anthranilic acid. For the purposes of the present invention the fluorescent label is available as an isotopic pair, containing a first stable isotope (e.g. light isotype) of a fluorescent label and a second stable isotope (e.g., heavy isotype) of a fluorescent label. Examples for such isotopic pairs useful in the present invention is isotopic 2-AA, for example, the stable isotopic variants ¹²[C₆]-2-AA and ¹³[C₆]-2-AA (Prien, J.M., et al., Analytical Chemistry 82, 1498-508 (2010)). Glycans labeled with such isotopic variants of for example 2-AA may be analyzed using positive-mode matrix-assisted laser desorption ionization time-of-flight mass spectrometry (MALDI-TOF MS) or on-line negative-mode electrospray mass spectrometry (ESI-MS/MSⁿ) and/or nanospray mass spectrometry (NSI-MS) (Prien, J.M., et al., Analytical Chemistry 82, 1498-508 (2010)). Other examples of a stable isotopic pair of a fluorescent label are ¹²[C₆]-aniline/ ¹³[C₆]-aniline and ¹²[C₆]-2-AB/¹³[C₆]-2-AB.

Carbohydrate moieties are described herein with reference to commonly used nomenclature for oligosaccharides. A review of carbohydrate chemistry which uses this nomenclature can be found, for example, in Hubbard and Ivatt, Ann. Rev. Biochem. 50, 555-583 (1981).

For the purposes of the present invention, a "glycan" refers to any sugar or assembly of sugars (*i.e*., saccharide or carbohydrate), in free form or attached to another molecule, such as proteins or lipids. As referred to herein, an "N-glycan" is a glycan covalently linked to an asparagine residue of a polypeptide chain in the consensus sequence: -Asn-X-Ser/Thr (*e.g*., comprising the common core structure Manα1-6(Manα1-3)Manβ1-4GlcNAcβ1-4GlcNAcβ1-N-Asn). As referred to herein, an "acidic glycan" is an N-glycan containing at least one terminal sialic acid (at the non-reducing terminus). As referred to herein, a "neutral glycan" is an N-glycan that does not contain any sialic acid. As referred to herein, "glycosylation" is the enzyme-catalyzed covalent attachment of a carbohydrate to a polypeptide, lipid, polynucleotide, carbohydrate, or other organic compound, generally catalyzed by glycosyltransferases, utilizing specific sugar nucleotide donor substrates. For the purposes of the present invention, a "polysaccharide" is a glycan composed of repeating monosaccharides, preferably greater than ten monosaccharide units in length. As referred to herein, a "sugar" refers to any carbohydrate, preferably to low molecular weight carbohydrates that are sweet in taste (see glossary of Essentials of Glycobiology. 2nd edition. Varki A, Cummings RD, Esko JD, et al., editors. Cold Spring Harbor (NY): Cold Spring Harbor Laboratory Press; 2009). The following abbreviations for monosaccharides are used: N-acetylglucosamine (GlcNAc), fucose (Fuc), mannose (Man), galactose (Gal), and sialic acid (SA).

For the purposes of the present invention, the term "glycoprotein" refers to peptides and proteins, including antibodies and fusion proteins (e.g., Fc-fusion proteins), having at least one glycan side chain.

The term "recombinant glycoprotein" as used herein refers to a glycosylated protein that results from the expression of recombinant DNA within a living cell. Recombinant DNA molecules are DNA molecules formed by laboratory methods of genetic recombination, such as molecular cloning, to bring together genetic material from multiple sources, creating sequences that would not otherwise be found in biological organisms. In the present context, the recombinant glycoprotein also includes that the protein is not endogenous to the mammal of which the liquid sample was obtained. In other words, the recombinant glycoprotein has been administered to the mammal prior to obtaining the sample. Preferred recombinant glycoproteins are therapeutic recombinant glycoproteins that have been expressed in higher eukaryotic cells. In this regard, exemplary useful higher eukaryotic cells are selected from the following cell lines:

| Cell Line | Meaning | Origin | Tissue Origin | Morphology |
|---|---|---|---|---|
| CHO | Chinese hamster ovary | Hamster | Ovary | Epithelium |
| COS-7 | *Cercopithecus aethiops,* origin-defective SV-40 | Ape - *Cercopithecus aethiops (Chlorocebus)* | Kidney | Fibroblast |
| BHK-21 | Baby hamster kidney fibroblast cells | Hamster | Kidney | Fibroblast |
| HEK-293 | Human embryonic kidney | Human | Kidney (embryonic) | Epithelium |
| HeLa | "Henrietta Lacks" | Human | Cervical cancer | Epithelium |
| HL-60 | Human leukemia | Human | Myeloblast | Blood cells |
| HUVEC | Human umbilical vein endothelial cell | Human | Umbilical vein endothelium | Epithelial |
| Jurkat | | Human | T cell leukemia | white blood cells |
| MCF-7 | Michigan Cancer Foundation-7 | Human | Mammary gland | Invasive breast ductal carcinoma |
| NIH-3T3 | NIH, 3-day transfer, inoculum 3 x 10⁵ cells | Mouse | Embryo | Fibroblast |
| RenCa | Renal carcinoma | Mouse | | Renal carcinoma |
| U937 | | Human | Leukaemic monocytic lymphoma | |
| Vero cells | *Vero* (truth) | African green monkey | Kidney epithelium | |

Particularly preferred in this regard are CHO cells or CHO derived cells (e.g., CHO-DXB11 or, CHO-DG55, which are widely used in the art to express biopharmaceutically useful glycoproteins, such as antibodies.

The glycoproteins may be homologous to the host cell, or may be heterologous, *i.e*., foreign, to the host cell being utilized, such as, for example, a human glycoprotein produced by a Chinese hamster ovary (CHO) host cell. In certain embodiments, the glycoproteins expressed by a host-cell are directly secreted into the medium.

Examples of suitable mammalian, and in particular human, glycoproteins include the following molecules: a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); a serum albumin, such as human serum albumin; mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-I (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

A preferred glycoprotein in the context of the present invention is a fusion protein. The term "fusion protein" as used herein refers to a chimeric protein containing two proteins or protein fragments fused to each other (i.e., expressed as one polypeptide) often separated by an amino acid linker, preferably an effector domain fused to an Fc domain, albumin or transferring. Thus, the fusion protein is preferably an Fc-fusion protein, a transferrin-fusion protein or an albumin-fusion protein, more preferably, the fusion protein is an Fc-fusion protein. Typically the Fc-domain in a fusion protein contains the CH2 and CH3 region and the hinge region of the IgG heavy chain, preferably of the IgG1 heavy chain. Fusion to an Fc-domain, albumin or transferrin usually increases the *in vivo* half-life and/or increases solubility of the effector domain. The amino acid linker may further contain a cleavage site for an endoproteinase. The effector domain may be a full length therapeutically relevant protein or a fragment thereof, particularly the extracellular part of a therapeutically relevant receptor or a membrane protein. Non limiting examples of therapeutically relevant Fc-fusion proteins are LFA-3/Fc (Alefecept), TNFR-Fc (Etanercept), CTLA-4/Fc (Abatacept and Belatacept), VEGFR1/2-Fc (Aflibercept), rFVIIIFc and rFIXFc, IL-1R1-Fc (Rilonacept), thrombopoietin-Fc (Romiplostim) and angiopoietin-1/2 antagonist peptide-Fc (Trebananib).

Examples of suitable mammalian, and in particular human, effector proteins suitable to be used in fusion proteins include the following molecules: a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-I (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

Another preferred glycoprotein in the context of the present invention is an antibody. The term "antibody" as referred to herein includes whole antibodies and any antigen binding fragment (i.e., antigen-binding portion) or single chain thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g*., effector cells) and the first component (C1q) of the classical complement system. Typically antibodies are glycosylated at the CH2 domain at asparagine residue N297. A significant number of antibodies also possess additional glycosylation sites (i.e., the Asn-X-Ser/Thr tripeptide) in their variable regions and N-linked glycosylation can be found in variable (V) domains of both heavy (VH) and light (VL) chains of serum IgG and of some monoclonal antibodies (mAbs). Examples of antigen-binding fragments encompassed within the term "antigen-binding portion" of an antibody include (i) a Fab fragment, a monovalent fragment consisting of the V_{L}, V_{H}, CL and CH1 domains; (ii) a F(ab')2 fragment, a bivalent fragment comprising two Fab fragments linked by a disulfide bridge at the hinge region; (iii) an Fd fragment consisting of the V_{H} and CH1 domains; (iv) an Fv fragment consisting of the V_{L} and V_{H} domains of a single arm of an antibody, (v) a dAb fragment (Ward et al., Nature. 341:544-546 (1989)), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g*., Bird et al. Science 1988, 242:423-426; and Huston et al., Proc. Natl. Acad. Sci. USA 1988, 85:5879-5883). Such single chain antibodies are also intended to be encompassed within the terms antigen-binding portion and antigen-binding fragment of an antibody. These antibody fragments are obtained using conventional techniques known to those with skill in the art, and the fragments are screened for utility in the same manner as are intact antibodies.

The term "monoclonal antibody" (mAb), as used herein, refers to an antibody which displays a single binding specificity and affinity for a particular epitope. Accordingly, the term "human monoclonal antibody" refers to an antibody which displays a single binding specificity and which has variable and constant regions derived from human germ line immunoglobulin sequences. In one embodiment, human monoclonal antibodies are produced by a hybridoma which includes a B cell obtained from a transgenic non-human animal, *e.g*., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (*e.g*., a mouse) that is transgenic or transchromosomal for human immunoglobulin genes or a hybridoma prepared therefrom, (b) antibodies isolated from a host cell transformed to express the antibody, *e.g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, a "heterologous antibody" is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

The term "peptide" as used herein refers to chains of amino acid monomers linked by peptide amide bonds. Peptides may be short chains of about 10 amino acids or less. Peptides may also be long chains of about 70 amino acids or more or anything in between. In the context of the present invention the fragments of a recombinant glycoprotein released by an endoproteinase is referred to as a peptide or glycopeptide if it carries a glycan.

As used herein "affinity ligand" refers to a ligand that binds specifically to the recombinant glycoprotein of interest. This affinity ligand is used to separate the recombinant glycoprotein of interest from the other compounds in the mammalian liquid sample, such as serum or plasma, using affinity chromatography. Typically, if the recombinant glycoprotein is an antibody, the affinity ligand is an antigen. For other recombinant proteins, the affinity ligand is typically a binding partner or a substrate of said recombinant protein. If the recombinant protein is a fusion protein the affinity ligand is typically a binding partner or a substrate of the effector domain of the fusion protein, e.g,, the ligand for a receptor or *vice versa.* Alternatively the affinity ligand may be an antibody specific for said recombinant protein or an antibody specific for said effector domain of the fusion protein. Preferably, the affinity ligand is aglycosylated, i.e., carries no glycans or at least no N-glycans, particularly if the glycans of the immobilized glycan containing fragment of the recombinant glycoprotein is further analyzed. The skilled person will understand that Protein A and Protein G or any other generically Fc-domain binding protein is not suitable as an affinity ligand for a specific recombinant Fc-fusion protein or antibody of interest in a liquid samples of a mammal containing endogenous antibodies, particularly for serum and plasma.

The term "antigen" as uses herein is a substance which provokes an adaptive immune response and may also be referred to as immunogen. An antigen binds to the antigen-binding site of an antibody. Antigens are typically of high molecular weight and proteins or polysaccharides. As used herein an antigen may also refer to the immunogenic part of an antigen comprising the epitope, e.g., peptides. Peptides, lipids, nucleic acids and many other materials can also function as antigens. Immune responses may also be generated against smaller substances, called haptens, if these are chemically coupled to a larger carrier protein, such as bovine serum albumin, keyhole limpet hemocyanin (KLH) or other synthetic matrices.

As used herein, "specific binding", "selective binding" and "selectively binds" and "ligand specific for", refer to an affinity ligand, binding to a predetermined recombinant glycoprotein, such as the effector domain of a fusion protein or to an antibody. For example, in one embodiment, the ligand binds with an affinity (KD) of approximately less than 10-7 M, such as approximately less than 10-8 M, 10-9 M or 10-10 M or even lower to the recombinant glycoprotein when determined by surface plasmon resonance (SPR) technology in a BIACORE 3000 instrument, and binds to the predetermined recombinant glycoprotein with an affinity that is at least two-fold greater than its affinity for binding to a non-specific antigen or other glycoproteins in the liquid sample. Thus, in the context of the invention "a ligand specific for" means that the ligand preferentially binds to the recombinant glycoprotein and thus separates said recombinant glycoprotein from other glycoproteins present in the sample, particularly from other antibodies present in the sample. The phrases "an antibody recognizing an antigen" and "an antibody specific for an antigen" are used interchangeably herein with the term "an antibody which selectively binds to an antigen" and may equally be used vice versa that "an antigen is specific for an antibody".

The term "eluate" as used herein relates to the material released from the solid support. It may comprise the buffer or solution used during elution or a different buffer or solution.

As used herein, "LC" as used herein means liquid chromatography, which may be combined with mass spectrometry, referred to as "LC-MS" herein. LC-MS may further include tandem MS. The term "reversed phase LC" or "HPLC" (RP-LC or RP-HPLC) as used herein has a non-polar stationary phase and an aqueous, moderately polar mobile phase, *e.g*., a silica which has been surface-modified with RMe₂SiCl, where R is a straight chain alkyl group such as C₁₈H₃₇ or C₈H₁₇ (Lehto and Hou, Chemistry and Analysis of Radionuclides, Wiley-VCH Verlag & Co., Weinheim, Germany, 2011, page 170). In the context of the present invention "reverse phase LC-MS" is preferred as the mobile phase used in reverse phase is more compatible with MS and therefore more sensitive than normal phase LC-MS.

As used herein "nano-LC" or nano-HPLC (RP-nano-LC or RP-nano-HPLC) is characterized by a decreased inner diameter of the columns that are used for LC (10-150 µm) and smaller flow-rates (10-1000 nl/min) compared to conventional LC or HPLC, respectively. This down-scaling results in high plate counts of the nano-LC system and the ability to analyze proteinaceous samples in the low femtomole and subfemtomole ranges (Chervet et al., Analytical Chemistry 1996, 68:1507-12). Consistent with the understanding and common general knowledge in the field of liquid chromatography, it will be appreciated that in accordance with the invention nano-LC and nano-HPLC are suitable and intended forms of LC and HPLC, respectively, for the purposes of the present invention; and that RP-nano-LC and RP-nano-HPLC are suitable and intended forms and even preferred forms of RP-LC and RP-HPLC, respectively. The same applies to the likewise well-known and established techniques of *micro-LC* and *capillary-LC,* or *RP-micro-LC* and *RP-capillary-LC,* which for the purposes of the present invention are suitable and intended forms of LC, and RP-LC, respectively. Where the terms LC, HPLC, LC-MS or HPLC-MS is used herein, this also encompasses their preferred embodiments, nano-LC, nano-HPLC, nano-LC-MS or nano-HPLC-MS and their reverse phase forms.

For the purposes of the present invention, a "mobile phase" of RP-LC or RP-HPLC is preferably a gradient of an organic modifier (*e.g*., acetonitrile or methanol) in water, with an ionic modifier that controls the pH and ionization state or acts as an ion pairing reagent. Anionic ion-pair reagents (*e.g*., trifluoroacetic acid (TFA)) bind to protonated basic groups of peptides. The addition of 0.1% TFA acidifies the eluent which causes the carboxylic groups of peptides and proteins to become protonated, resulting in a larger hydrophobicity of the molecules. Cationic ion-pairing reagents (*e.g*., triethylammonium ions) bind to ionized carboxyl groups of peptides (*"*Protein Liquid Chromatography", Journal of Chromatography Library, vol. 61, edited by Kastner M, Elsevier Science B.V., 2000, page 153). Diethylamine (DEA) can also be used as an ion pairing reagent (Melmer et al., Journal of Chromatography A (2011), Volume: 1218(1): 118-123). For normal phase or HILIC chromatography of e.g. 2-AB labeled N-glycans, a suitable mobile phase consists for example of 60 mMol ammonium formate in 75% acetonitrile (mobile phase A) and 115 mMol ammonium formate in 54% acetonitrile (mobile phase B) (Melmer et al., Anal Bioanal Chem (2010), Volume 398: 905-914).

As used herein, "ion trap mass spectrometry" is an arrangement in which ions with a desired range of quotients mass/charge are first made to describe stable paths under the effect of a high-frequency electric quadrupole field, and are then separated and presented to a detector by adjusting the field so as to selectively induce path instability according to their respective mass/charge ratios *e.g*., quadrupole ion trap (see http://www.genomicglossaries.com/content/mass spectrometry.asp). Sensitivity of the methods of the invention can be improved by using more sensitive nano ESI sources.

For the purposes of the present invention, all "N-glycans" are understood to have the common core sugar sequence, Manα1-6(Manα1-3)Manβ1-4GlcNAcβ1-4GlcNAcβ1-Asn-X-Ser/Thr, and are classified into three types: (1) "oligomannose" (high mannose), in which only mannose residues are attached to the core; (2) "complex", in which "antennae" initiated by N-acetylglucosaminyltransferases (GlcNAcTs) are attached to the core; and (3) "hybrid", in which only mannose residues are attached to the Mana1-6 arm of the core and one or two antennae initiated by N-acetylglucosaminyltransferases (GlcNAcTs) are on the Mana1-3 arm (Essentials of Glycobiology, 2nd edition, Varki et al., editors, Cold Spring Harbor (NY): Cold Spring Harbor Laboratory Press; 2009; Chapter 8).

For the purposes of the present invention, a reference to the analysis of acidic or neutral glycans, N-glycans, or glycans in general, "in one run", "in one approach" or "in a single (analytical) approach" means that the MS analysis is directly coupled to (or in other words, is performed on-line with) the LC or RP-LC step. This means that there is no further analytical or preparatory step between the RP-LC and the MS analysis. It will be understood that this does not exclude that fluorescence detection will occur between the RP-LC and the MS. Indeed, fluorescence detection of the 2-AA labeled glycans separated on the RP-column will normally (and advantageously) occur before they leave the LC system and proceed to MS, since the fluorescence detector (FLD) that is passed by the labeled glycans is typically a module of the HPLC/LC system. This is also referred to as "LC-MS" or RP-LC-MS".

The term "enzymatic cleavage" as used herein refers to any cleavage of a glycoprotein that involves an enzyme and allows for release of the recombinant glycoprotein while avoiding elution of most non-specifically bound glycoproteins. The enzymatic cleavage is done using an endoproteinase. The enzyme should be selective for the recombinant glycoprotein of interest. This means that the specific cleavage site is rare and preferably only present in the recombinant glycoprotein of interest or additionally in a few other glycoproteins, such as a specific family of glycoproteins, or a group of glycoproteins all comprising the same domain. A more selective enzyme avoids elution of fragments of non-specifically bound glycoproteins from the solid support. Preferably the enzyme cleaves the recombinant glycoprotein efficiently and specifically (e.g., only at a known cleavage site). Suitable enzymes are for example endoproteinases that cleave within the recombinant glycoprotein of interest specifically at a certain consensus sequence and that have narrow substrate specificity (or high selectivity). Preferably, the endoproteinase is specific (and/or selective) for the recombinant glycoprotein of interest or a domain contained in the recombinant glycoprotein of interest. For example, a suitable endoproteinase specifically only cleaves the recombinant glycoprotein of interest or a group of proteins comprising essentially the same domain, including the recombinant glycoprotein of interest. Examples for a group of proteins comprising essentially the same domain are antibodies and Fc-fusion proteins, all containing an Fc-domain. A suitable enzyme has preferentially only one cleavage site in the recombinant glycoprotein of interest or within each identical polypeptide chain within the recombinant glycoprotein of interest. The terms "endopeptidase", endoproteinase", "proteinase" or "protease" are used interchangeably herein.

Preferably, the released glycan containing fragment is a peptide containing one glycan or in the case of a released Fc-domain two identical peptides containing one glycan each. For example endoproteinases suitable in the methods of the invention to release the glycan containing Fc-domain of the recombinant glycoprotein from the solid support are endoproteinase, such as papain, ficin, cysteine protease SpeB (FabULOUS) or cysteine proteinase IdeS (FabRICATOR®), preferably the endoproteinase is the cysteine proteinase IdeS (FabRICATOR®). For example IdeS specifically cleaves human IgG in the hinge region between the two glycines of the constant sequence ELLGGPS and SpeB cleaves in the hinge region between threonine and cysteine within the sequence KTHTCPPC. A glycanase may have specificity for certain *N*- or *O*-glycosylation sites. Such a glycanase is for example EndoSGlycanase (IgGZERO®), which is specific for N-glycans in IgG Fc-domain containing glycoproteins, which are cleaved after the first GlcNAc at the reducing end.

Alternatively, fusion proteins such as Factor IX-albumin (FIX-albumin) comprise a linker sequence between the two domains that is based on amino acids 137-153 derived from the N-terminus of the activation peptide of FIX. Activation of FIX-recombinant albumin fusion protein by either FXIa or FVIIa/TF cleaves the linker, thereby separating the FIXa and rHA moieties of the fusion protein. Thus, FXIa and/or FVIIa/TF are suitable to cleave FIX-albumin selectively. Also Factor Xa is a site-specific protease that exhibit very low non-specific cleavage under many conditions. Thus, Factor Xa may be used for cleaving glycoproteins that contain a Factor Xa cleavage site (cleavage behind arginine of the sequence Ile-Glu/Asp-Gly-Arg). Factor Xa kits are e.g. available from Novagen (69036-3). Likewise thrombin is a site-specific protease for specific cleavage between arginine and glycine of the sequence LeuValProArgGlySer in recombinant fusion proteins.

The term "solid support" as used herein refers to any solid surface that can be used to immobilize a ligand thereon, particularly in affinity chromatography. Typically a "solid support" suitable for affinity chromatography is a resin, such as a resin comprising microbeads with a large surface area. Particularly suitable resins in the present invention are sepharose beads, agarose beads or magnetic beads, wherein sepharose beads are preferred. The term "immobilized" refers to covalent or non-covalent binding to the solid support, either directly or indirectly. Typically the affinity ligand is covalently coupled to the solid support, for example via N-hydroxysuccinimide (NHS), cyanogen bromide, epoxide, carboiimide or thiopropyl reactive groups. Commercially available microbeads activated with one of the above reactive groups are known in the art, e.g., NHS-activated sepharose. Once the recombinant glycoprotein binds to its specific affinity ligand immobilized or coupled to the solid support the recombinant glycoprotein is immobilized on the solid support via non-covalent binding to the affinity ligand.

The term "liquid sample of a mammal" as used herein refers to any liquid sample obtained from a mammal at a certain time point comprising biological material such as cells, protein, DNA or RNA. Typically the liquid sample is a body fluid, such as serum or plasma. However, the liquid sample may also be whole blood, urine, cerebral spinal fluid, amniotic fluid, saliva, sweat, ejaculate, tears, phlegm, vaginal secretion, vaginal wash or colonic wash. Preferably the sample is cleared from cells or debris, e.g., by centrifugation prior to be subject to the methods of the invention. For the liquid sample to contain the recombinant glycoprotein of interest, the sample must have been obtained following administration of said recombinant glycoprotein to the mammal. In the context of the present invention the recombinant glycoprotein is to be understood as a biopharmaceutical or therapeutically active recombinant glycoprotein. The skilled person will understand that a liquid sample, or an "aliquot" of a liquid sample, may be used in the present invention. If only an aliquot of the liquid sample is used to be analyzed according to the methods of the present invention, other aliquots of the same sample may be analyzed for different parameters, such as protein concentration, or stored frozen for later analysis.

The term "lower limit of quantification (LLOQ)" as used herein refers to the lowest concentration of an analyte in a sample that can be quantitatively determined with suitable precision and accuracy", wherein the analyte is the recombinant glycoprotein. The LLOQ is typically given as µg/ml. the skilled person understands that it is therefore strongly dependent on the sample volume used for analysis.

The term "reference standard" as used herein refers to a standardized mixture of glycans with a known relative distribution labeled with an isotopic variant of a fluorescent label different to the isotopic variant used for labeling the glycans to be analyzed. Isotope labeling can be introduced into glycans at the reducing end, for example using a stable (preferably heavy) isotopic variant of the fluorescent label, e.g. ¹³[C₆]-2AA. The mixture of glycans contains at least the glycan structures to be analyzed. The mixture is further standardized, which means that the identical mixture of glycans is added to each sample of the experiment or to each sample that is compared to each other. Preferably, the glycans of one batch of the recombinant glycoprotein are released, labeled and stored frozen until use.. The use of a reference standard compensates variations in the sample preparation and nanoLC-MS analysis resulting in more precise results. It is therefore preferably added as early as possible to the sample. Moreover, the reference standard allows for analyzing the glycan structures individually. This means the amount of each glycan structure can be quantified individually relative to the respective glycan structure of the reference standard based on the known relative distribution of the individual glycans in the reference standard, rather than the relative proportion of the glycans within each sample. Hence, PK parameter of individual glycan structures can be determined.

The term "high throughput" as used herein relates to a mode or method that permits rapid and highly parallel sample preparation of a number of samples, such as more then 10, more than 50, more than 100, more than 500 or more than 1000 samples.

For the purposes of the present invention, a reference to % will be understood to refer to (v/v) unless explicitly stated otherwise.

As used herein, the term "about" when used together with a numerical value (e.g., a pH value or a percentage value) is intended to encompass a deviation of 20%, preferably 10%, more preferably 5%, even more preferably of 2%, and most preferably of 1% from that value. When used together with a numerical value it is at the same time to be understood as individually disclosing that exact numerical value as a preferred embodiment in accordance with the present invention.

As used herein, the term "comprising" is to be construed as encompassing both "including" and "consisting of", both meanings being specifically intended, and hence individually disclosed, embodiments in accordance with the present invention.

Accordingly, the present invention relates to a method of analyzing glycans of a recombinant glycoprotein of interest in liquid samples of a mammal comprising a) immobilizing the recombinant glycoprotein of interest comprised in each of two or more liquid samples on a separate solid support coupled to an affinity ligand specific for the recombinant glycoprotein in the samples; b) releasing a glycan containing fragment of the recombinant glycoprotein of each of said samples from the solid support into separate eluates by enzymatic cleavage of the recombinant glycoprotein using an endoproteinase; c) optionally releasing the glycans from the glycan containing fragment of the recombinant glycoprotein of each of said samples in the separate eluates; d) labeling the glycans of each of said samples with a first stable isotope of a fluorescent label; and e) analyzing the labeled glycans of step d) of each of said samples separately using LC-MS and comparing said two or more samples, wherein a reference standard comprising glycans labeled with a second stable isotope of the fluorescent label is added to each of said samples prior to step b), c), d) or e) and the reference standard is analyzed together with the labeled glycans of step e). It is preferred that the reference standard is added early during the process. Thus, the reference standard may be added prior to step e), preferably prior to step e) or prior to step c) and even more preferably prior to step b). All steps of the method of the invention are performed separately for each of the two or more liquid samples obtained from a mammal.

The present invention also relates to a method further comprising analyzing the glycans of a second glycan containing fragment of the recombinant glycoprotein of each of said samples that remains immobilized to the solid support in step b), wherein the recombinant glycoprotein is an Fc-fusion protein or an antibody, comprising the following additional steps: aa) pre-clearing each of the two or more liquid samples of a mammal to remove glycoproteins binding non-specifically to the solid support prior to step a), comprising aai) immobilizing Fc-containing glycoproteins on a solid support using an Fc-binding protein, wherein said Fc-binding protein is preferably selected from protein G or protein A. more preferably said Fc-binding protein is protein G; and aaii) eluting the Fc-containing glycoproteins: wherein the solid support used in said pre-clearing step is made of the same material as the solid support used in step a) of claim 1, but is not coupled to said affinity ligand; cc) releasing the glycans from the immobilized second glycan containing fragment of the recombinant glycoprotein of each of said samples remaining on the solid support following step b) into separate solutions; eedd) labeling the glycans of each of said samples with a first stable isotope of a fluorescent label; and ee) analyzing the labeled glycans of step eedd) of each of said samples separately using LC-MS and comparing said two or more samples, wherein a reference standard comprising glycans labeled with a second stable isotope of the fluorescent label is added to each of said samples prior to step cc), dd) or ee) and wherein the reference standard is analyzed together with the labeled glycans of step ee). The pre-clearing step aa) may comprise contacting the samples with a solid support, wherein the solid support is made of the same material as the solid support used in step a), but is not coupled to said affinity ligand. It is preferred that the reference standard is added early during the process. Thus, the reference standard may be added prior to analyzing the labeled glycans using LC-MS (step ee), preferably prior to the labeling of the glycans to be analyzed (step dd) and more preferably added on-column prior to release of the glycans of the effector domain (step cc). In cases where the glycans of the second glycan containing fragment of the recombinant glycoprotein of each of said samples that remain immobilized to the solid support in step b) are further analyzed, preferably no reference standard has been added to each of said samples prior to step b). The skilled person will understand that all steps of the method of the invention are performed separately for each of the two or more liquid samples obtained from a mammal.

Preferably, the fluorescent labeling is done after the glycans have been released from the glycoprotein fragment. Release of the glycans may be done chemically or enzymatically by methods well known to those skilled in the art.

Affinity purification from liquid samples of a mammal, such as a serum samples, may require a pre-clearing step. A pre-clearing step is particularly advantageous, if the glycans of the immobilized second glycan containing fragment following enzymatic cleavage are to be analyzed. The pre-clearing step may comprise contacting the samples with a solid support, wherein the solid support is made of the same material as the solid support used for immobilizing of the recombinant glycoprotein (step a) without the coupled affinity ligand. Non-specifically binding glycoproteins or glycolipids remain bound to the solid support. Thus, the recombinant glycoprotein may be in the flow through, i.e., the material not binding to the solid support. Alternatively, the pre-clearing step may be an upstream second affinity purification step immobilizing the recombinant glycoprotein on the solid support followed by eluting the recombinant glycoprotein from the solid support.

In another aspect the invention relates to a method of analyzing glycans of an Fc-fusion protein containing an Fc-domain and a effector domain of interest in liquid samples of a mammal, comprising aa) pre-clearing each of two or more liquid samples of a mammal comprising: immobilizing the Fc-fusion protein of interest comprised in each of said two or more liquid samples on a separate solid support using an Fc-binding protein, wherein said Fc-binding protein is preferably selected from protein G or protein A, more preferably said Fc-binding protein is protein G; and eluting the Fc-fusion protein; a) immobilizing the Fc-fusion protein of each of said samples on a separate solid support coupled to an affinity ligand specific for the effector domain of the Fc-fusion protein in the sample, wherein the affinity ligand is a binding partner or an antibody specifically binding to the effector domain of the Fc-fusion protein; b) releasing the Fc-domain of the Fc-fusion protein of each of said samples from the solid support into separate eluates by cleaving with an endopeptidase specific for Fc-fusion proteins; cc) releasing the glycans from the immobilized effector domain of the Fc-fusion protein of each of said samples remaining on the solid support following step b) into separate solutions; dd) labeling the glycans of each of said samples with a first stable isotope of a fluorescent label; andf ee) analyzing the labeled glycans of step dd) of each of said samples separately using LC-MS and comparing said two or more samples, wherein a reference standard comprising glycans labeled with a second stable isotope of the fluorescent label is added to each of said samples prior to step cc), dd) or ee) and wherein the reference standard is analyzed together with the labeled glycans of step ee).

According to the methods of the present invention the glycans to be analyzed are preferably N-glycans. N-glycans to be analyzed may be high mannose type, hybrid type or complex type N-glycans. Alternatively the glycans to be analyzed may also be O-glycans.

The fluorescent label as used in the methods of the invention may be any label suitable for LC-MS analysis such as 2-amino benzamide (2-AB), 2-amino benzoic acid (2-AA) or 8-aminonachthalene-1, 3, 6-trisulfonic acid (ANTS). However, there must be a stable light and a stable heavy isotope variant available to label the glycans to be analyzed and the reference standard with a different isotope variant of the same fluorescent label. Preferably, the fluorescent label is a ¹²[C] and ¹³[C] isotopic pair, more preferably a ¹²[C6] and ¹³[C6] isotopic pair, and even more preferably a ¹²[C₆]-2-aminobenzoic acid (¹²[C₆]-2-AA) and a ¹³[C₆]-2-aminobenzoic acid (¹³[C₆]-2-AA) isotopic pair.

It will be appreciated that the labeling with 2-AA in accordance with the methods of the present invention is particularly advantageous for mapping (*i.e*., analyzing) neutral or acidic glycans of a recombinant glycoprotein, such as an antibody or a fusion protein. The glycans to be analyzed are preferably N-glycans, particularly N-glycans that are either of the oligomannose, hybrid or complex type. In one embodiment the N-glycans are G1F isomers, particularly the G1F isomers with 1,3 or 1,6 galactosylation.

The reference standard used in the methods according to the invention comprises glycans labeled with a stable (second) isotope of the fluorescent label, preferably with a stably heavy isotope of the fluorescent label. Preferably, the reference standard comprises at least the same glycan structures as analyzed in the sample. If the glycans to the analyzed are in the form of glycan containing fragments of the recombinant glycoprotein, such as glycopeptides, the reference standard equally comprises glycans in the form of the glycan containing fragment of the recombinant glycoprotein, such as glycopeptides. The use of a reference standard compensates variations in the sample preparation and nanoLC-MS analysis, resulting in more precise results. It is therefore preferably added as early as possible to the sample. Moreover, the reference standard allows for analyzing the glycan structures individually as the samples can be normalized. This means the amount of each glycan structure can be quantified individually relative to the respective glycan structure of the reference standard based on the known relative distribution of the individual glycans in the reference standard. Hence, PK parameter of individual glycan structures can be determined. The reference standard may be prepared by releasing the glycans of the recombinant glycoprotein of interest and labeling the glycans with one of the stable isotope variants of the fluorescent label. The reference standard may be the same or different in steps d) and dd) in the present invention. Preferably the reference standard in step d) and dd) is the same. For example for analyzing the glycans of a specific fusion protein both the reference standard in steps d) and dd) may comprise glycans released from said full-length fusion protein. Alternatively the reference standard in step d) may comprise the glycans released from the fusion partner, such as the Fc-domain, albumin or transferrin, of the fusion protein and the reference standard in step dd) may comprise the glycans released from the effector domain, such as the extracellular domain of TNFR, of the fusion protein. However, the reference standard must contain the glycan structure of the glycans to be analyzed and the relative proportion of the glycan structures within the glycans of the reference standard should be known. Using a reference standard as described above allows for determining the relative amount of each glycan structure separately.

The recombinant glycoprotein analyzed in the methods of the invention is preferably a fusion protein or an antibody, more preferably an Fc-fusion protein or an antibody. In embodiments where the recombinant protein is an Fc-fusion protein or antibody the glycan containing fragment of the recombinant glycoprotein released from the solid support in step b) is preferably an Fc domain.

For Fc-domain containing glycoproteins, the method of the invention may comprises the following steps: a) immobilizing the recombinant glycoprotein of interest having an Fc-domain comprised in each of two or more liquid samples on a separate solid support coupled to an affinity ligand specific for the recombinant glycoprotein in the samples; b) releasing a glycan containing fragment of the recombinant glycoprotein of each of said samples, preferably the Fc-domain, from the solid support into separate eluates by enzymatic cleavage of the recombinant glycoprotein; c) optionally releasing the glycans from the Fc-domain of the recombinant glycoprotein in the separate eluates; d) labeling the glycans of each of said samples with a first stable isotope of a fluorescent label; and e) analyzing the labeled glycans of step d) of each of said samples separately using LC-MS and comparing said two or more samples, wherein a reference standard comprising glycans labeled with a second stable isotope of the fluorescent label is added to each of said samples prior to step b), c), d) or e) and the reference standard is analyzed together with the labeled glycans of step e).

In one particularly preferred embodiment, the recombinant glycoprotein is an antibody and the variable region of the antibody binds to the affinity ligand immobilized on the solid support, preferably the affinity ligand is an antigen. In a preferred embodiment the antibody is an IgG antibody selected from IgG1, IgG2, IgG3 or IgG4, more preferably an IgG1 or IgG2 antibody. The antibody may be a chimeric, human or humanized IgG antibody, preferably the antibody is a human or humanized IgG1 or IgG2 antibody.

In another particularly preferred embodiment the recombinant glycoprotein is an Fc-fusion protein comprising an Fc-domain and an effector domain and the effector domain binds to the affinity ligand immobilized on the solid support, wherein the affinity ligand is a binding partner or an antibody specifically binding to the effector domain of the Fc-fusion protein.

When the glycans of the second glycan containing fragment of the recombinant glycoprotein of each of said samples immobilized to the solid support are further analyzed, a pre-clearing step of the liquid sample is required. Preferably, the pre-clearing step is a second affinity chromatography upstream of the immobilization of the recombinant glycoprotein in step a), enriching the recombinant glycoprotein in the sample following elution. Preferably, recovery rate of the recombinant protein of interest is high, e.g., 90% or more, 95% or more, 98% or more, 99% or more or 100 %. For Fc-domain containing glycoproteins, an Fc-binding protein may be used for enriching the recombinant glycoprotein. For Fc-domain containing glycoproteins the pre-clearing step preferably comprises aai) immobilizing Fc-containing glycoproteins on a solid support using an Fc-binding protein, wherein the solid support is made of the same material as the solid support used for immobilizing the recombinant glycoprotein in step a) without the coupled affinity ligand used in step a) and aaii) eluting the Fc-containing glycoproteins. Preferably the Fc-binding protein is protein G or protein A, more preferably said Fc-binding protein is protein G. After binding to the Fc-binding protein and intensive washing, IgGs or Fc-fusion proteins are eluted. Suitable methods to elute IgGs and fusion proteins from Fc-binding proteins are known to the person skilled in the art, and include without being limited thereto the use of acidic conditions, such as glycine or arginine buffer of low pH. Due to the typically lower affinity of protein G (or protein A) to the Fc-domain compared to the binding of an antibody to its antigen or of a therapeutic fusion protein to its target, the recovery is almost complete. Non-specifically bound endogenous glycoproteins that could falsify the analysis remain bound to the solid support. The pre-cleared sample is neutralized and added to an affinity column with immobilized affinity ligand.

For Fc-fusion proteins following immobilization and release of the Fc-fusion protein by enzymatic cleavage (steps a and b), the glycans of the effector domain of the Fc-fusion protein remaining on the solid support, are released into a solution. The glycans are labeled with a fluorescent label following glycan release and analyzed together with the reference standard. The reference standard comprising glycans labeled with a second stable isotope of the fluorescent label may be added on-column prior to release of the glycans of the effector domain (step cc), prior to the labeling of the glycans to be analyzed (step dd) or prior to analyzing the labeled glycans using LC-MS (step ee).

Preferably the reference standard comprises N-glycans from a mixture of effector domain and Fc-domain glycans labeled with a stable isotope variant of the fluorescent labeled, such as a stable isotope variant of 2-AA.

In one embodiment the recombinant protein is an antibody or an Fc-fusion protein and the pre-clearing step comprises aai) immobilizing the Fc-containing glycoproteins on a solid support using an Fc-binding protein, wherein said Fc-binding protein is preferably selected from protein G or protein A, more preferably said Fc-binding protein is protein G; and aaii) eluting the Fc-containing glycoproteins; wherein the solid support used in said pre-clearing step is made of the same material as the solid support used in step a), but is not coupled to said affinity ligand.

The enzymatic cleavage according to the methods of the invention, releasing the glycan containing fragment of the glycoprotein is done using an endoproteinase. Preferably the endoproteinase cleaves the recombinant glycoprotein efficiently and specifically (e.g., only at a known cleavage site). More preferably the enzyme is further selective for the recombinant glycoprotein of interest. This means that the specific cleavage site is rare and only present in the recombinant glycoprotein of interest and few other glycoproteins, such as a specific family of glycoproteins, or a group of glycoproteins all comprising the same domain, such as Fc-containing glycoproteins. A more selective enzyme avoids or reduces elution of fragments of non-specifically bound glycoproteins from the solid support. Preferably, the released glycan containing fragment is a peptide containing one glycan or in the case of a released Fc-domain two peptides containing one glycan each. For example the endoproteinases suitable in the methods of the invention to release the glycan containing Fc-domain of the recombinant glycoprotein from the solid support are endoproteinase, such as papain, ficin, cysteine protease SpeB (FabULOUS) or cysteine proteinase IdeS (FabRICATOR®), preferably the endoproteinase is the cysteine proteinase IdeS (FabRICATOR®). IdeS specifically cleaves human IgG in the hinge region between the two glycines of the constant sequence ELLGGPS. Enzymatic cleavage using endoproteinases is typically very efficient, resulting in almost complete release of the glycan containing glycoprotein fragment. Compared to acidic elution of therapeutic antibodies or fusion proteins immobilized via their respective ligand (using for example glycine of arginine), elution via enzymatic cleavage is often more efficient and complete and therefore results in more sensitivity of the method of the invention. Non-selective endoproteinases, such as Lys-C that hydrolyses specifically at the carbonyl side of Lys or trypsin, cleaving several times within most glycoproteins are not well suited in the context of the present invention.

Glycanases may have specificity for certain *N*- or *O*-glycosylation sites. Such a glycanase is for example EndoSGlycanase (IgGZERO®), which is selective for the N-glycan in Fc-domain containing glycoproteins. However, it cleaves GlcNAc-β1,4-GlcNAc bonds in the N-glycan of the Fc-domain and therefore releases a glycan lacking fucose. Thus, EndoSGlycanase is only suitable where fucosylation is not of interest.

In fusion proteins, N-glycans of the effector domain and the fusion partner, such as an Fc-domain, albumin or transferrin can be analyzed separately to gain more information about site specificity. In the following the method according to the invention will be exemplified for an Fc-fusion protein. However, the skilled artisan knows how to adapt the method to other fusion proteins. For individual analysis of the Fc part and effector domain N-glycans the two parts have to be separated. The fusion protein is therefore immobilized with its effector domain on a solid support, such as a sepharose resin and the Fc part is released enzymatically using an enzyme such as IdeS enzyme. IdeS is an endopeptidase which selectively cleaves IgGs and related molecules with high specificity below the hinge region producing a Fab2 and Fc fragments. IdeS also cleaves IgG Fc-domain containing fusion proteins. The fusion protein is cleaved into an effector domain (e.g., receptor part), which is connected by disulfide bridges and able to bind the interaction partner or antigen and an Fc-domain. Alternatively any other endoproteinase could be used that cleaves selectively between the glycans of the effector domain and the glycans of the Fc-domain. Alternatively for other fusion proteins suitable endoproteinases cleave between the glycans of the effector domain and the glycans of the fusion partner, such as albumin or transferrin. Preferably, the endoproteinase only cuts the fusion protein between the glycans of the effector domain and the glycans of the Fc-domain. Even more preferably, the endoproteinase selectively cuts the fusion protein or a group (or family of proteins including the fusion protein) within the sample, but not all proteins within the sample. This provides further specificity of the method and reduces contamination with fragments of non-specifically bound glycoproteins. For example, the IdeS enzyme cleaves IgG Fc-domains below the hinge region in antibodies and Fc-fusion proteins and therefore reduces contamination of the released Fc-domain by other contaminating glycoproteins that do not have an Fc-domain. The fusion protein may also contain a specific cleavage site, which has been introduced by molecular cloning between the effector domain and the fusion partner, which may be used in this method.

The fluorescent label as used in the methods of the invention may be any label suitable for LC-MS analysis such as 2-amino benzamide (2-AB), 2-amino benzoic acid (2-AA) or 8-aminonachthalene-1, 3, 6-trisulfonic acid (ANTS). However, there must be a stable light and a stable heavy isotope variant available to label the glycans to be analyzed and the reference standard with a different isotope variant of the same fluorescent label. Preferably, the fluorescent label is a ¹²[C] and ¹³[C] isotopic pair, more preferably a ¹²[C6] and ¹³[C6] isotopic pair, and even more preferably a ¹²[C₆]-2-aminobenzoic acid (¹²[C₆]-2-AA) and a ¹³[C₆]-2-aminobenzoic acid (¹³[C₆]-2-AA) isotopic pair.

It will be appreciated that the labeling with 2-AA in accordance with the methods of the present invention is particularly advantageous for mapping (*i.e*., analyzing) neutral or acidic glycans of a recombinant glycoprotein, such as an antibody or a fusion protein. The glycans to be analyzed are preferably N-glycans, particularly N-glycans that are either of the oligomannose, hybrid or complex type. In one embodiment the N-glycans are G1F isomers, particularly the G1F isomers with 1,3 or 1,6 galactosylation.

Following fluorescent labeling of the glycans in the methods of the present invention, access label may be separated using gel filtration. For example Sephadex G10 columns may be equilibrated with water and the mixture comprising fluorescently labeled glycans, free fluorescent label and optionally the reference standard is added to the gel filtration column. The labeled glycans and the reference standard are eluted with water following by evaporating the water in a vacuum centrifuge and the dried sample may be dissolved in water for LC-MS analysis. The skilled person will understand that the gel filtration can be performed with many samples in parallel by using multiwell plates such as 96 well plates or other sizes.

Release of the glycans from the glycan containing fragment of the recombinant glycoprotein can be done chemically or enzymatically by methods well known to those skilled in the art.

For example, chemical release of glycans for subsequent labeling (or analysis, as the case may be) may be affected by hydrazinolysis or by alkaline β-elimination, methods that are well known to those of skill in the art. An exemplary useful kit for chemical removal is the GlycoProfile™ IV Chemical Deglycosylation Kit offered by Sigma-Aldrich.

Release of the glycans by enzymatic methods is preferred and causes no problems to those of skill in the art. A particularly preferred method of glycan removal for subsequent labeling (or analysis) is digestion with PNGaseF (Peptide N-glycosidase F). Various suitable PNGaseF enzymes are offered commercially under different trade names (*e.g*., N-Glycanase®), which are mostly engineered or optimized PNGaseF, although using an engineered or optimized PNGaseF is not mandatory in the context of the present invention. Enzymatic release may also be done by using Endoglycosidase H (or an enzyme with similar enzymatic activity, such as EndoSGlycanase IgGZERO™) or Endoglycosidase F2, which cleave between the two N-Acetylglucosamines of the glycan core leaving the first monosaccharide attached to the protein. However, the information whether the glycan carried a fucose or not is lost in this way. Furthermore Endoglycosidase H and Endoglycosidase F2 are only specific for oligomannose and hybrid bi-antennary glycans.

For glycan analysis in liquid samples of a mammal, such as serum, on-column deglycosylation should only be used in combination with a pre-clearing step prior to step a), because endogenous serum glycoproteins that bind non-specifically to the sepharose resin and cannot be removed by additional washing steps. With only one N-glycosylation site at the Fc part, antibodies are particularly suited for an enzymatic elution step. The enzyme IdeS selectively cleaves IgG heavy chains C-terminal of the disulfide connection thereby releasing the two glycosylated heavy chain fragments that can subsequently be deglycosylated with high efficiency. Theoretically more than one enzymatic elution step can be performed after one another. For glycan analysis of the immobilized glycan containing fragment of the recombinant glycoproteins, on-column deglycosylation is performed in combination with a pre-clearing step prior to immobilization of the recombinant glycoprotein to the solid support (step a)). Preferably, the pre-clearing step comprises affinity purification to enrich the recombinant glycoprotein in the sample.

The LC-MS used in the methods of the present invention is preferably a reverse phase LC-MS or a NanoLC-MS, more preferably the LC-MS is a reverse phase NanoLC-MS.

Methods for affinity purifying antibodies, fusion proteins or other glycoproteins are known to the skilled person. The solid support used may be a resin, such as a resin comprising microbeads, preferably sepharose beads, agarose beads or magnetic beads, more preferably sepharose beads. However any solid support or resin suitable for affinity chromatography can be used in the methods of the present invention. The affinity ligand may be coupled to the solid support via N-hydroxysuccinimide (NHS), cyanogen bromide, epoxy, carbodiimide or thiopropyl, preferably via N-hydroxysuccinimide (NHS). Again, methods for coupling affinity ligands to a solid support are known in the art. Typically resins activated with one of the above linkers are commercially available.

The recombinant glycoproteins that will typically be analyzed using the methods of the present invention are therapeutically relevant recombinant glycoproteins. Therapeutically relevant recombinant glycoproteins often have a very high affinity and specificity to its therapeutic target. Hence the therapeutic target is well suited as an affinity ligand to capture the recombinant glycoprotein from the liquid sample of a mammal and immobilize it to the solid support. For example affinity purification of an antibody with the respective antigen has the advantage of very high affinity and specificity due to the strong interaction of the antibody with its antigen. The affinity of a therapeutic antibody for its antigen is typically in the picomolar to low nanomolar range. Therapeutically relevant fusion proteins, such as etanercept, typically bind their therapeutic target with a similar affinity. However, one major drawback of this high affinity binding is the limited recovery of antibodies or fusion proteins after acidic elution. Thus, by eluting a glycan containing fragment of the glycoprotein using enzymatic cleavage the recovery may be improved.

The affinity ligand may be any binding partner, including without being limited thereto the ligand for a receptor, the substrate, an antigen or an antibody. For therapeutically relevant glycoproteins the affinity ligand is preferably the therapeutic target or a fragment thereof. The affinity ligand may also be a mutant of the therapeutic target or a fragment thereof, preferably a mutant binding to the recombinant glycoprotein with higher affinity compared to the wild type therapeutic target or fragment thereof. Where the recombinant glycoprotein is an antibody, the affinity ligand is preferably an antigen binding to the antibody, more preferably an antigen binding to the antibody with high affinity. The antigen may be for example a complex of more than one proteins, a full length protein or a fragment thereof, including a short peptide. For fusion proteins the affinity ligand may be a binding partner preferably binding to the effector domain of the fusion protein. The affinity ligand may also be an antibody binding to the effector domain of a fusion protein. For any other glycoprotein of interest, the affinity ligand may likewise be a binding partner or an antibody binding to the recombinant glycoprotein of interest. Preferably, the affinity ligand is not glycosylated.

In a preferred embodiment of the methods of the invention the two or more liquid samples of a mammal or aliquots thereof are prepared for analysis using a multi well filter plate, preferably a 24, 96 or 384 well filter plate, more preferably a 96 well filter plate. The filter membrane is preferably a low protein binding and/or hydrophile membrane, such as nitrocellulose or polyvinylidene difluoride (PVDF) membranes. These filter plates are suitable for affinity chromatography, enzymatic digest, fluorescent labeling and gel-filtration, depending on the reagents or resins added.

The liquid sample of a mammal comprising the recombinant protein is a body fluid and is preferably selected from the group consisting of serum or plasma, urine, cerebral spinal fluid, amniotic fluid, saliva, sweat, ejaculate, tears, phlegm, vaginal secretion, vaginal wash and colonic wash; preferably said sample is a plasma or a serum sample. Preferably the samples to be analyzed have a volume from about 1 µl to about 1000 µl, from about 5 µl to about 500 µl, from about 10 µl to about 200 µl, from about 10 µl to about 100 µl, from about 25 µl to about 100 µl, or from about 40 µl to about 75 µl, preferably of about 50 µl. In a preferred embodiment the two or more liquid samples of a mammal were obtained from the same subject. The mammalian liquid sample analyzed in the methods of the invention may be a human, a monkey, a rodent, a dog, a cat or a pig sample, preferably a rodent sample such as from mouse, rat, hamster or rabbit.

The methods of the present invention may be used to determine pharmacokinetic parameters of at least one specific glycan structure of the glycans of the recombinant glycoprotein of interest, preferably the Cₘₐₓ, tₘₐₓ, AUC or t_{1/2}. Preferably the two or more liquid samples of a mammal were obtained at different time points from the same subject, wherein at least about 24 h, 48 h, 72 h, 96 h, 120 h, 144 h, 168 h, 336 h, 504 h, 672 h, 840 h may be between the first and the last time point. Preferably the two or more liquid samples of a mammal are 5 or more, 10 or more, 20 or more, 30 or more, 40 or more or 50 or more samples.

The methods of the invention may further comprise the steps of (i) determining the area under the curve of the extracted ion chromatograms (EICs) of the glycans from each of said samples labeled with the first stable isotope of the fluorescent label and of its respective reference standard glycan labeled with the second isotope of the fluorescently label to form a ratio of first isotope sample glycan to second isotope reference standard over time, and (ii) optionally calculating glycan percentages based on the known relative distribution of the individual glycans in the reference standard for each of said samples.

Further, the ratios or the glycan percentages of the two or more samples for each individual glycan may be compared, e.g., plotted against time. Preferably the glycans are N-glycans.

For example the L/H ratios of the sample N-glycans (light signal) to the constant heavy isotope standard N-glycans (heavy signal) plotted against time result in glycan PK profiles for each N-glycan. Alternatively calculated sample N-glycan percentages may be plotted against time, resulting in glycan PK profiles of each N-glycan. Determined glycan PK profiles can be compared against the respective ELISA profile and are the basis for calculation of the glycan maps. The skilled person will understand that the stable light isotope could equally be used as a label in the reference standard, when the stable heavy isotope is used for labeling the glycans to be analyzed. In other words, as long as the glycans of the reference standard are labeled with a fluorescent label of a different isotype variant as the glycans to be analyzed, the stable light and the heavy isotope fluorescent label are equally suited. The skilled person will understand that the percentages (%) of the glycans refer to mol, because the "heavy" glycan reference standard composition is determined using the UV signal, which depends solely on the label which is directly proportional to the number of labeled glycan molecules.

The glycans may also be analyzed according to the methods of the invention in an aliquot of each of said two or more liquid samples of a mammal. Preferably the aliquots to be analyzed have a volume from about 1 µl to about 1000 µl, from about 5 µl to about 500 µl, from about 10 µl to about 200 µl, from about 10 µl to about 100 µl, from about 25 µl to about 100 µl, or from about 40 µl to about 75 µl, preferably of about 50 µl. Optionally, the concentration of said recombinant glycoprotein may be analyzed in a further aliquot of said two or more liquid samples of a mammal. In one embodiment, the concentration of said recombinant glycoprotein is analyzed by ELISA or any other method known to the person skilled in the art to determine concentrations of a specific protein in a sample.

The liquid chromatography-MS to be performed in connection with the methods of the present invention is preferably reverse phase liquid chromatography (RP-LC-MS), more preferably reversed-phase high performance liquid chromatography (RP-HPLC-MS), or ultra performance liquid chromatography (UPLC-MS). In this case, as in other preferred cases of RP-LC performed in connection with the present invention, these chromatography methods are performed on a reversed-phase liquid chromatography column. Preferably the RP-LC is performed under conditions under which the carboxy group of the 2-AA label attached to the glycans is neutral. Reverse phase LC is preferred in the methods of the present invention as it allows more sensitive analysis compared to normal phase LC, because the mobile phase is more compatible with mass spectrometry. Optionally, no ion-pairing reagent is used in the mobile phase. Preferably, an acidic mobile phase is used for the RP-LC.

The liquid chromatography to be performed in connection with the methods of the present invention is preferably a nano-LC or nano-HPLC, even more preferably a reverse phase nano-LC or a reverse phase nano-HPLC. Nano-LC is characterized by a decreased inner diameter of the columns that are used for LC (10-150 µm) and smaller flow-rates (10-1000 nl/min) compared to conventional LC or HPLC, respectively. This down-scaling results in the ability to analyze proteinaceous samples in the low femtomole and subfemtomole ranges (Chervet et al., Analytical Chemistry 1996, 68:1507-12) and therefore allows to reduce the sample volume to be analyzed and the duration to detect glycans of recombinant glycoproteins post administration

A suitable mobile phase used during RP-LC comprises formic acid. In this regard, preferred amounts of formic acid in the mobile phases are from about 0.1% to about 2.0% formic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% formic acid. An amount of about 1.0% formic acid in the mobile phase is particularly preferred.

Another suitable mobile phase used during RP-LC comprises acetic acid. In this regard, preferred amounts of acetic acid in the mobile phases are again from about 0.1% to about 2.0% acetic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% acetic acid. An amount of about 1.0% acetic acid in the mobile phase is particularly preferred.

Further, suitable pH values of the mobile phase used during RP-LC are in the range of about 1 to about 4, more preferably in the range of about 1.5 to about 3, yet more preferably of about 1.8 to about 2.9, even more preferably of about 1.9 to about 2.75, and particularly preferably of about 2 to about 2.7. Preferred is in particular a pH value in the range of about 2.1 to about 2.18. Accordingly, preferred mobile phases used during RP-LC in accordance with the methods and uses described and/or claimed herein will have a pH value of about 1.9, 2, 2.1, 2.2, 2.3, 2.4, 2.5, 2.6, or 2.7, with pH values of about 2.1 or of about 2.18 being particularly preferred.

In the course of the methods of the present invention the separation of the fluorescent (preferably 2-AA) labeled glycans by LC or RP-LC may be advantageously performed at temperatures in the range of about 4°C to about room temperature, or at room temperature (with room temperature being defined as 23°C for the purpose of the present invention). Preferably, however, the separation is performed at temperatures above room temperature. Preferred in this regard is a temperature in the range of about 40°C to about 60°C, with about 50°C being particularly preferred.

Suitable flow rates for a nanoRP-LC or nanoLC column in accordance with the present invention will be readily known or determined by those of skill in the art. Generally, suitable flow rates will typically be in a range of about 50-1000 nl per minute. Preferred are, for example, flow rates in a range of about 100-500 nl per minute. Accordingly, preferred values for the flow rate are about 100 nl per minute, about 200 nl per minute, about 300 nl per minute, about 400 nl per minute, or about 500 nl per minute, with a flow rate of about 300 nl per minute being particularly preferred.

As noted above, the methods of the present invention comprise subjecting the fluorescently labeled glycans (preferably 2-AA labeled glycans) to mass spectrometry (MS) after their separation via LC or RP-LC, with methods where this is done by directly coupling LC or RP-LC with the MS analysis being particularly preferred. Mass spectrometry methods suitable in this regard include ion-trap mass spectrometry, such as positive ionization mass spectrometry.

In a preferred embodiment the methods of the invention allow for analyzing attomolar concentrations of the individual labeled glycans, e.g., concentrations as low as 800 amol, preferably as low as 600 amol, and more preferably as low as 400 amol. In another preferred embodiment the method allow for analyzing the glycans of the recombinant glycoprotein at a recombinant glycoprotein concentration in each of the two or more liquid samples of a mammal of 20 µg/ml or less, 10 µg/ml or less, 5 µg/ml or less, 2 µg/ml or less, 1 µg/ml or less, 0.5 µg/ml or less, 0.2 µg/ml or less or 0.1 µg/ml or less. In yet another preferred embodiment the methods of the present invention allow for analyzing the glycans of the recombinant glycoprotein in in the two or more liquid samples of a mammal comprising 1.0µg or less, 0.5 µg or less, 0.25 µg or less, 0.1 µg or less, 0.05 µg or less, 0.025 µg or less, 0.01 µg or less, or 0.005 µg or less of the recombinant protein each. In yet another preferred embodiment at least steps b), c), d) and e) and/or at least steps aa), dd) and ee) are operated in a high throughput manner.

The present disclosure also relates to a method of preparing a glycoprotein based pharmaceutical composition comprising analyzing the glycans of a recombinant glycoprotein in liquid samples of a mammal of according to any one of the preceding claims; and formulating the glycoprotein into said pharmaceutical composition. Optionally the recombinant glycoprotein is glycoengineered prior to formulating the glycoprotein into the pharmaceutical composition.

The invention is further illustrated by the following Figures and Examples, which are not to be considered as being limiting for the scope of protection conferred by the claims of the present application.

### Examples

### Materials

2-Aminobenzoic acid, ethanolamine, formic acid, picoline borane, DMSO, ¹³C aminobenzoic acid were from Sigma (Munich, Germany). PNGaseF was from Roche (Penzberg, Germany). Acetic acid, acetonitrile and hydrochloric acid were from Merck (Darmstadt, Germany). Protein G, NHS activated sepharose, Sephadex® G-10 96-well plates and 96-well deep well plates were from GE Healthcare (Munich, Germany). TNF-alpha was from Peprotech (Hamburg, Germany). Phosphate buffered saline was from Gibco/Life technologies (Darmstadt, Germany).

Multicreen THS HV filter plates were from Milipore. Fabricator was Genovis (Lund, Sweden). 96-well plates were from Nunc/Thermo Scientific (Munich, Germany). AcroPrep™ Advance Omega™ 10K 96-well filter plates were from Pall (Dreieich, Germany). Pre-clinical rabbit serum samples were obtained from clinical bioanalytics at Sandoz.

### Example 1: Influence of glycol-variants on the pharmacokinetics of an IgG1 biopharmaceutical

### Preclinical rabbit study

The preclinical study was performed in New Zealand White rabbits. Following single subcutaneous administration of 10 mg kg-1 body weight of an IgG1 mAb1 (anti-TNF-a antibody) blood samples were drawn over a period of time including one pre-dose blood sample. Serum samples were taken at 12 time points after administration. Detailed sampling is listed in Table 1. Concentration of mAb1 in serum was determined by ELISA. From remaining serum 2 x 50 µl aliquots were used for glycan PK profiling. The first aliquot was analyzed and the second aliquot served as back-up aliquot.

**Table 1: Sampling schedule of the pre-clinical study of an IgG1. At each sampling time point ∼500 µl of serum were drawn.**

| Day | 1 | 1 | 1 | 2 | 2 | 3 | 3 | 4 | 5 | 8 | 15 | 22 | 29 |
|---|---|---|---|---|---|---|---|---|---|---|---|---|---|
| Hours post-dose | 0 (pre-dose) | 2 | 8 | 24 | 40 | 48 | 60 | 72 | 96 | 168 | 336 | 504 | 672 |

### Reconstitution of the antigen

Recombinant human antigen (TNF-α) produced in E.coli was reconstituted according to the manufacturer instructions. Antigen was dissolved in H₂O (1 mg/mL) and reconstituted for 2 hours at room temperature.

### Preparation of ¹³C 2-AA labeled glycan standard

N-glycans of desalted mAb (1 mg) were released using PNGaseF digest overnight (17 h) at 37°C. The N-glycans were separated from the proteins by use of Amicon 30K filter devices and were brought to dryness using a speedvac. Picoline borane and [¹³C] 2-AA were dissolved in 70:30(% v/v) DMSO-acetic acid to furnish concentrations of 63 and 50 mg/ml, respectively. Labeling solution (15 µL) and deionized water (10 µL) were added to 15 nmol enzymatically released and dried glycans. The labeling reaction was performed at 37°C for 17 h.

Excess label was removed by gel filtration on G-10 columns. Columns were conditioned with 10 ml H₂O. Samples were diluted to 100 µl with deionized water and applied to the column. After rinsing the column with 700 µl H₂O the purified fluorescence labeled N-glycans were eluted with 600 µl H₂O. Purified [¹³C] 2-AA labeled N-glycans are aliquoted and stored at -20°C until use.

### Preparation of 96-well plate affinity columns with immobilized antigen

The membranes of a 96 well filter plate comprising a hydrophilic low-protein binding membrane (Multiscreen THS HV filter plates) were wetted with 1mM HCl (100 µL) before addition of 200 µL NHS activated sepharose-isopropanol slurry per well. Isopropanol was removed by centrifugation and the columns were washed with 1 mM HCl (150 µL) for four times. Antigen solution (100 µL; 50 µl/ml) was centrifuged into the columns and coupling reaction was allowed to take place for 2 hours at ambient temperature. Affinity columns were washed and remaining NHS groups were inactivated using ethanolamine buffer (150 µL). Finally, columns were equilibrated with PBS.

### Affinity purification of an IgG1 biopharmaceutical and glycan release

Serum samples (50 µL) were diluted to 100µL with PBS and applied to the affinity purification column by centrifugation. Bound mAb was washed several times with PBS to remove serum and non-specific bound proteins. Fabricator solution (1 U/µl) was centrifuged into the columns to release the glycosylated Fc part of the mAb. Reaction was performed at 37°C for 30 minutes. Released Fc parts were eluted with PBS and PNGaseF with ¹³C-2-AA labeled N-glycan standards was added. This mixture was incubated for 17 hours at 37°C. Remaining proteins were removed by ultrafiltration using 96-well plates with 10 K cut-off membranes. Released N-glycans with glycan standard were dried by vacuum centrifugation.

### N-qlycan labeling

Dried samples containing free reducing end N-glycans and ¹³C 2-AA labeled glycan standard were dissolved in H₂O (10 µL), 2-AA labeling solution (15 µL; 100 mg/mL picoline borane, 50 mg/mL 2-AA in 7:3 mixture of DMSO and acetic acid) was added and incubated for 17 hours at 37°C.

### Gel filtration

Excess label is subsequently removed by gel filtration. Custom made 96-well plate Sephadex G-10 columns were equilibrated with 800 µL H₂O. Labeled samples were filled up to 100 µL with H₂O and applied to the gel filtration columns. 2-AA and ¹³C 2-AA labeled N-glycans were eluted with H₂O (150 µL). Finally samples were brought to dryness by vacuum centrifugation and were dissolved in 20 µL H₂O for nanoLC-MS analysis.

### NanoLC of labeled N-glycans

Labeled sample N-glycans and the heavy isotope standard were analyzed by RP nanoLC-MS. NanoLC (Thermo/Dionex Ultimate 3000) was set-up in "pre-concentration" mode according to the manufacturer manual with a pre-concentration column (3 µm particles, 75 µm x 2 cm) and an analytical column (2 µm particles, 75 µm x 25 cm). Column compartment was held at 40°C. Mobile phase of the nano pump consisted of 0.5% formic acid in H₂O (component A) and 0.5% formic acid in 50% ACN (component B). Mobile phase of the capillary pump consisted of 0.5% formic acid and 1% ACN in H₂O (component C). The analytical column was equilibrated with 2% component B at a flow rate of 300 nl/min. Pre-concentration column was equilibrated with 100% component C. With a user defined injection routine, 8 µl sample were stacked between loading solution (0.1% formic acid, 1% ACN in ultrapure water) in a 20 µl sample loop. Sample loop was switched for 2 minutes in-line of the capillary pump flow to allow optimal trapping. Prior to the next injection sample the loop was washed with loading solution. After trapping the pre-concentration column was switched into the nano pump flow and component B was raised to 30% over 60 minutes, then to 95% over 5 minutes. After holding at 95% component B for 5 minutes the column was finally re-equilibrated at 2% component B for 15 minutes. Column outlet was connected to a UV detector with a 3 nl flow-cell.

### Mass Spectrometry

The outlet of the nanoLC was directly coupled to an ion trap ESI-MS (Bruker AmaZon) equipped with an on-line nano source (Bruker CaptiveSpray®). The ion trap was operated in Enhanced Resolution Mode with a capillary voltage of 1.7 kV. Source temperature was set to 200°C and a dry gas flow of 3 l/min was used to heat the source.

Data interpretation is done by determining the area of the respective light sample glycans and its appropriate heavy isotope standard from their EICs. This light to heavy ratio (L/H) is determined for each N-glycan at each time point and animal. By plotting the L/H ratio against time PK profiles for each N-glycan are obtained. The N-glycan percentages can be calculated with the known relative distribution of heavy isotope standard glycans for each time point.

### mAb1 N-glycosylation and qualification of the study

To qualify and control the affinity purification, QC samples were prepared by spiking known amounts of mAb1 into NZW rabbit serum. The QC samples covered the concentration range of the study which was previously determined by ELISA. Figure 2A shows the mean percentages of the mAb1 QC samples. The samples covered the concentrations between 10 µg/ml and 100 µg/ml. In total four QC samples (0 µg/ml, 1 µg/ml; 10 µg/ml and 100 µg/ml) in duplicates were used including one serum blank. The mean percentages of the glycans obtained from the QC samples represent the mAb1 N-glycan composition. With the developed enzymatically elution of the glycosylated antibody fragments very high selectivity and purity could be achieved. No interfering N-glycans of mAb1 were co-purified with the exception of two minor abundant bisecting variants that were excluded from analysis. No additional serum related N-glycans were detected. Sensitivity was at least sufficient to analyze all N-glycans with a percentage of at least 0.1 % at a concentration of 10 µg/ml (LLOQ). However, sensitivity can be improved to about 0.1 µ/ml using improved nano ESI sources.

The N-glycans was mainly complex biantennary with core fucose (Figure 2B). As may be taken from Figure 2A, the most abundant N-glycan was G0F (65 %) with terminal N-acetylglucosamine residues followed by G1F (16 %) with one additional terminal galactose. High mannose glycan M5 (9.5 %) was the third most abundant N-glycan. All other N-glycans had a percentage of less than 3%. mAb1 contained no N-glycans with terminal sialic acids.

### Preclinical study: Glycan PK profiles of mAb1 and ELISA

The determined glycan L/H ratios were plotted against the sampling time to obtain PK profiles for each N-glycan. Mean L/H ratios for each N-glycan were normalized to the maximum. ELISA data were normalized in the same way. The relative concentrations of the ELISA and L/H values were then compared graphically. Figure 3 shows the resulting curves of the N-glycans. Mean ELISA profiles are depicted in diamonds with error bars representing the variability. The most abundant complex type G0F had a very similar PK profile compared to the ELISA profile (Figure 3A). The maximal concentration (tₘₐₓ) was achieved after 72 h with congruency of the two graphs. At the beginning of elimination there was a slight difference, however, with complete overlap of the error bars the PK profiles can be regarded comparable. This finding was expected for the major N-glycan with almost 70 % relative abundance, as the ELISA represents the average profile of all glycoforms to which the most abundant N-glycan contributes most.

The second most abundant complex type N-glycan G1F that accounts for approximately 16 % showed also a very similar PK profile when compared to the ELISA profile (Figure 3B). Again tₘₐₓ was reached at 72 h for both curves.

Complex type G2F with a percentage of 2 % had the best match with the ELISA profile showing almost perfect congruency (Figure 3C). tₘₐₓ was reached earlier with 60 h compared to 72 h of the ELISA curve. Due to the very small difference between the 60 h and 72 h time point and the fact that there was no additional sampling point, the two profiles can still be regarded identical.

The profile of the M6G0F/M5G1F hybrid type glycan, which are two isomers that could not be differentiated with the nanoLC-MS approach and had a relative content of only 0.1 %, still looks similar to the ELISA profile (Figure 3D). The low abundance was close to the LLOQ which resulted in lower precision of the L/H values than for the other more abundant glycans. However, from the similar tₘₐₓ and the similar curves it can be concluded that the PK was identical or at least similar.

These results demonstrate that PK profiles can be obtained for each N-glycan individually. The glycan PK profiles were highly similar to the ELISA for the most abundant glycans. Merely for N-glycans with a portion smaller than 0.5 % the graphs were not that clearly due to the higher variation as a result of the small abundance.

The glycan PK profiles of the high mannose type N-glycans M5 and M6 showed a completely different picture. The curves of the N-glycan with 9.5 % and 2 % relative abundance for M5 and M6, respectively are shown in Figure 4. M6 had a PK profile that deviated extremely from the average profile determined by ELISA (Figure 4A). The maximum concentration was achieved after 24 h followed by either a conversion to M5 or an increased elimination rate that lead to almost complete removal from circulation.

High mannose glycan M5 also had a different PK profile when compared to ELISA (Figure 4B). tₘₐₓ was reached 24 h earlier after 48 h and furthermore clearance was faster between 48 h and 168 h. These results demonstrate that high mannose glycan PK profiles of mAb1 were different in comparison to the ELISA which represents the average concentration of all protein variants including glyco-variants. These findings are also reflected in the glycan maps that were determined for each time point (see Figure 5) to strengthen the theory of a conversion and increased clearance of high mannose glycans. PK profiles of M3G1F, M3G0F and the core structure M3 are shown in (Figure 3E-G). PK profiles for GO could not be obtained due to co-elution of a contaminant with the same m/z value.

### Selective clearance of M5 and M6

The composition of mAb1 N-glycosylation is shown in Figure 2. Based on the known relative amount of the heavy isotope glycan standard and the experimentally obtained L/H ratios the percentage of each N-glycan was calculated. Figure 5 shows the resulting glycan maps of the preclinical study. Glycan maps were calculated for time points between 8 h and 336 h. At 2 h, 504 h and 672 h the serum concentration of mAb1 was below 10 µg/ml for many rabbits, which is the LLOQ of this study, and therefore no glycan maps could be calculated.

Mean percentages of the most abundant N-glycans G0F and G1F stayed constant, which confirm previously observations (Figure 5 left). The magnified view shows the minor abundant glycans (Figure 5 right). The percentages stayed constant for all N-glycans except the high mannose glycans. M5 and M6 portion decreased over time. M6 was removed from circulation whereas the M5 portion decreased from the initial percentage of 9 % to approximately 4 %.

It is known from literature that high mannose species M6-M9 are converted to smaller high mannose glycans like M5 in humans (Chen et al., Glycobiology (2009) 21, 949-59; Alessandri et al., MAbs (2012) 4(4), 509-520). In mice a similar observation was made. High mannose species M7-M9 are converted to M6 (Yu et al., mAbs (2012) 4(4), 475-87). Both observations were made for *in vitro* incubation of antibody in serum. In the present study mainly M5 was removed selectively from circulation. An increase of smaller glycans like M4 or M3 (core structure) was not observed. A further conversion of M5 would require additional enzymes with other specificities in serum, because the linkage of the terminal mannose residues of M5 is different to that of M6-M9. It is very likely that M5 or rather M5 containing glycoproteins, are removed by a specific clearance mechanism from circulation e.g. through the mannose receptor.

**Table 2: Glycoforms containing M5.**

| | **M5:M5** | **M5:G1F** | **M5:M3G1F** | **M5:G0F** | **M5:M3G0F** | **G0F:G0F** |
|---|---|---|---|---|---|---|
| **Calculated relative*** | 0.62% | 1.12% | 0.11% | 5.29% | 0.18% | 45.43% |
| **Calculated relative to G0F:G0F** | 1.36% | - | - | 11.64% | - | 100% |
| **Observed relative to G0F:G0F** | 5.13% | - | - | 12.73% | - | 100% |

| | | | | | | |
|---|---|---|---|---|---|---|
| ^{*}Values are calculated based on the assumption of random pairing. | | | | | | |

The incomplete removal of M5 from circulation can be explained with the structural conformation of the Fc part. Several investigations showed that IgG glycoforms containing N-glycans with terminal galactosylation, which have a similar size as M5, change the conformation of the Fc part to an open conformation, a horseshoe conformation which makes the N-glycans accessible for other proteins to bind (Krapp et al., J. Mol. Biol. (2003), 325, 979-989). This implies that M5 pairs with N-glycans resulting in a glycoform that is large enough to force the Fc part into an open conformation. It was shown that pairing of the glycosylated heavy chains during protein biosynthesis is not random (Masuda et al., FEBS Lett. (2000), 473, 349-57. For an IgG2 biopharmaceutical it was demonstrated that glycoform M5:M5 is favored (Goetze et al., Glycobiology (2009) 19, 240-9). For mAb1 in the present study similar observations were made (Table 2). Assuming random pairing, all glycoforms can be easily calculated, which is shown for M5 containing glycoforms. MS data generated from intact Fc parts of mAb1 show that the M5:M5 glycoform was four times higher than the calculated value. M5:M5 was strongly favored during protein biosynthesis. It is likely that this glycoform results in a horseshoe conformation of the Fc part and in turn an increased clearance from circulation by binding to the Mannose receptor. The remaining 4 % M5 observed in the preclinical study could be the M5:G0F glycoform which would be small enough for a closed Fc conformation. This would explain the incompletely clearance from circulation.

### Conclusion

A new approach for the investigation of individual N-glycan pharmacokinetics was utilized to analyze a preclinical rabbit study of an IgG1 biopharmaceutical. Using 96 well plate based high throughput affinity purification with immobilized antigen and stable heavy isotope standard mass spectrometric quantification glycan PK data from 50 µl serum samples were obtained for mAb concentrations between 10 and 90 µg/ml. Glycan PK profiles were compared to ELISA data demonstrating that high mannose glycans M5 and M6 had a different PK profile. Glycan maps showed that M6 was removed during the first 48 h completely and M5 levels decreased from 9.5 % to approximately 4 %.

The results obtained from the preclinical study presented were similar to the results that were obtained from healthy human subjects by another group (Goetze et al., Glycobiology (2009) 19, 240-9). These findings demonstrate the value of glycan PK profiling with preclinical samples. The use of stable heavy isotope 2-AA glycan standards enables the PK profiling of individual N-glycans by mass spectrometry which was so far not described and enables an independent analysis of single N-glycans in comparison to so far used relative analysis of the N-glycans.

In summary, this new approach for glycan PK profiling with its high sensitivity works with preclinical samples. The results obtained from the preclinical study furthermore demonstrate that results from human subjects are identical with findings in preclinical studies. Implementation during earlier biopharmaceutical development allows the investigation of N-glyosylation effects already during preclinical studies. This allows implementation of preventive actions to optimize N-glycosylation, glyco-engineering, before entering clinical phases.

### Example 2: Influence of glycol-variants on the pharmacokinetics of a therapeutic fusion protein

### Preclinical rabbit study

The preclinical study was performed in Himalayan rabbits. Following single subcutaneous administration of 8 mg kg-1 body weight of two different batches of the Fc-fusion protein etanercept (FP1 or FP2), blood samples were drawn over a period of time including one pre-dose blood sample. Sampling was performed as listed in Table 3. At each sampling time point at least 600 µl of serum were drawn. Concentration of FP1 and FP2 in serum was determined by ELISA. From remaining serum 2 x 50 µl aliquots were used for glycan PK profiling. The first aliquot was analyzed subsequently and the second aliquot served as back-up aliquot.

### Preparation of ¹³C 2-AA labeled glycan standard

N-glycans of desalted FP1 fusion protein (1 mg) were released using PNGaseF digest overnight (17 h) at 37°C. The N-glycans were separated from the fusion protein by use of Amicon 30K filter devices and were brought to dryness using a speedvac. Picoline borane and [¹³C] 2-AA were dissolved in 70:30(% v/v) DMSO-acetic acid to furnish concentrations of 63 and 50 mg/ml, respectively. Labeling solution (15 µL) and deionized water (10 µL) were added to 15 nmol enzymatically released and dried glycans. The labeling reaction was performed at 37°C for 17 h.

Excess label was removed by gel filtration on G-10 columns. Columns were conditioned with 10 ml H₂O. Samples were diluted to 100 µl with deionized water and applied to the column. After rinsing the column with 700 µl H₂O, the purified fluorescence labeled N-glycans were eluted with 600 µl H₂O. Purified [¹³C] 2-AA labeled N-glycans are aliquoted and stored at -20°C until use.

### Preparation of 96-well plate affinity columns with immobilized Protein G

Protein G sepharose (200 µL) was added to each well of the 96-well filter plate comprising a hydrophilic low-protein binding membrane (Multiscreen THS HV filter plates). Protein G sepharose was stored in 20% ethanol which must be removed before affinity purification. Columns were therefore equilibrated with PBS (150 µL) for four times. Liquid was removed by centrifugation.

### Preparation of 96-well plate affinity columns with immobilized antigen

The membranes of a 96 well filter plate (Multiscreen THS HV filter plates) were wetted with 1mM HCl (100 µL) before addition of 200 µL NHS activated sepharose-isopropanol slurry per well. Isopropanol was removed by centrifugation and the columns were washed with 1 mM HCl (150 µL) for four times. Reconstitution of the antigen expressed in *E.coli* was performed as described in Example 1 above. Antigen solution (100 µL) was centrifuged into the columns and coupling reaction was allowed to take place for 2 hours at ambient temperature. Affinity columns were washed and remaining NHS groups were inactivated with use of ethanolamine buffer (150 µL). Finally columns were equilibrated with PBS.

### Affinity purification of a fusion protein and glycan release

Serum samples (50 µL) were added onto the equilibrated Protein G column of the 96 well plate and centrifuged through the column. The column was subsequently washed with PBS (150 µL) six times by centrifugation. Bound IgGs were eluted with three times 100 µL elution buffer (0.1 M glycine pH 2.7). Eluat was immediately neutralized with 1 M Tris HCl pH 8.0. The eluat contained the target protein as well as other IgGs from serum.

Eluat was added onto the equilibrated immobilized antigen column of the 96 well plate and centrifuged through the column. Column was then washed six times with PBS (150 µL). Fabricator solution (100 µl, 1 U/µl) was centrifuged into the columns to release the glycosylated Fc part of the fusion protein. Reaction was performed at 37°C for 30 minutes. Released Fc parts were eluted with PBS. PNGaseF with ¹³C-2-AA labeled N-glycan standards was added to the eluted Fc parts and onto the column with the antigen bound part. Digests were incubated for 17 hours at 37°C. From antigen bound protein part released N-glycans were eluted with H₂O. Remaining proteins were removed by ultrafiltration using 96-well plates with 10K cut-off membranes. Released N-glycans with glycan standard were dried by vacuum centrifugation

N-glycan labeling, gel filtration to remove excess 2-AA labeling reagent and analysis of labeled sample N-glycans and the heavy isotope standard by RP nanoLC-MS were performed as described in Example 1.

### Glycan maps of the two batches reveal different N-glycosylation

N-glycan PK profiling of monoclonal antibodies is usually less sophisticated than PK profiling of other biopharmaceuticals, because mAbs carry only one conserved N-glycosylation site on each heavy chain at the Fc part. It is therefore not necessary to analyze the N-glycans with site specificity. For more complex glycosylated biopharmaceuticals site specificity provides additional information that could help understanding the mechanism of clearance or prolonged half-life. For example fusion protein consists of two chains containing the CH2 and CH3 domain of an IgG Fc part and a receptor part. Like in mAbs the chains are connected by disulfide bridges. The Fc part carries one N-glycan per chain and the receptor part two additional N-glycosylation sites making a total of six N-glycans per molecule.

For individual analysis of the Fc part and receptor part N-glycans the two parts were separated using the endoproteinase IdeS enzyme and the generated fragments were analyzed individually. After separation Fc part and receptor part were deglycosylated using PNGaseF. The glycans were labeled with 2-AA and analyzed by nanoLCMS. The resulting glycan maps are shown in Figure 6.

The two fusion protein batches had a different N-glycosylation pattern. The glycosylation of fusion protein 1 (FP1) was more heterogenic whereas fusion protein 2 (FP2) has higher percentage of only few N-glycans. Both batches contained high percentages of terminal sialic acids at their receptor part. The found Fc glycosylation was typical for antibodies with a high portion of complex bi-antennary N-glycans and core fucosylation; some carried an additional sialic acid. Main difference between FP1 and FP2 were the terminal groups. FP1 had a high percentage of terminal GlcNAc residues. In contrast FP2 had a high degree of terminal galactosylation, while the sialylation was comparable.

### Average PK profiles of FP1 and FP2 in a preclinical study in rabbits

The preclinical study was conducted in Himalayan rabbits. After a single subcutaneous administration of FP1 or FP2, samples were taken and the concentration of FP1 and FP2 was determined by ELISA. The sampling schedule is depicted in Table 3. Five animals from each arm of the study were included for glycan PK profiling.

**Table 3: Sampling schedule of the pre-clinical study of FP1 and FP2. Prior to the administration a pre-dose sample was taken from each rabbit.**

| Day | 1 | 1 | 1 | 1 | 2 | 2 | 2 | 3 | 4 | 6 | 8 |
|---|---|---|---|---|---|---|---|---|---|---|---|
| Hours [post-dose] | 0 (pre-dose) | 2 | 6 | 12 | 18 | 24 | 32 | 48 | 72 | 120 | 168 |

The ELISA results of the five animals of both arms are shown in Figure 7. The PK profiles were different. FP1 had higher concentrations than FP2, but the error bars are overlapping. Both profiles reached their tₘₐₓ, the time point of the highest concentration, after 18 h. Clearance of FP1 was faster which results in congruency at 72 h.

The discrepancy of the ELISA profiles and the different N-glycosylation was investigating for a possible relationship.

### Comparison of individual N-glycan PK profiles with average ELISA data

Relative concentration of ELISAs was determined by normalizing average profiles to the maximal concentration. The relative concentration of the individual N-glycan profiles was determined by normalizing the average profiles, based on the determined L/H ratios, to the maximal L/H ratio. Figure 8 shows the average PK profiles of the major N-glycan of the Fc part (Figure 8A) and the receptor part (Figure 8B) of both fusion proteins FP1 and FP2 determined by nanoLC-MS (filled squares) compared to the appropriate average ELISA profiles (filled diamonds).

The variability of data of different animals was high which is reflected by the error bars, with a higher variability for the N-glycans located at the receptor part. N-glycan profiles of FP1 G0F, FP1 SG2F and FP2 G1F showed a shift in tₘₐₓ towards later time points compared to the respective ELISA. Only FP2 SG2F had an identical tₘₐₓ. This course indicates a prolonged serum half-life with a decreased clearance rate. However, glycan PK profiles of FP2 were closer to the ELISA profile than the glycan PK profiles of FP1.

### Glycan maps of FP1 and FP2

The glycan maps were obtained on the basis of the L/H ratios determined by nanoLC-MS. With the known distribution of the spiked stable heavy isotope 2-AA labeled N-glycan standard the glycan maps were calculated with high precision. The glycan maps of the Fc and receptor part from FP1 are shown in Figure 9. The N-glycans of the Fc part were mainly neutral complex biantennary N-glycans. Major glycans were G0F (open diamond) and G1F (filled triangle). All other N-glycans were below 10%. The percentages of the glycans of the FC part were constant over time.

The receptor part contains higher portions of acidic N-glycans. SG2F (open square) was the most abundant N-glycan. In contrast to the Fc part, the percentages of the receptor part changed over time. The portions of G0F (open diamond) and G2F (open triangle) decreased over time, whereas the portions of SG2F (open square) and SG2 (filled diamond) showed a trend towards higher percentages. The deviation at 32h (Fc part) was most likely due to variations during the affinity purification or nanoLC-MS analysis and no temporary change of the N-glycosylation pattern.

Similar results were obtained for FP2. The resulting glycan maps were shown in Figure 10. For the Fc part the glycan map show that the major N-glycans G1F (filled triangle), G2F (open triangle), G0F (open diamond) and SG2F (open square) and all minor abundant glycans had constant percentages. The receptor part showed a different picture. The major N-glycan SG2F (open square) increased slightly (relative to other glycans) and the N-glycan G2F (open triangle) decreased drastically over time (relative to other glycans).

Shifts of minor abundant N-glycans are discussed in the following. The variation observed between 6h and 12h at the Fc part was most likely due to the low protein concentration resulting in a higher variation and not due to a significant change of the N-glycan percentages.

### Terminal galactosylation of the receptor part accelerated clearance

N-glycans with terminal galactosylation (G1F and G2F) and the N-glycan G0F carry a terminal N-acetylglucosamine. G2F showed decreasing percentages over time for FP1 and FP2 (Figures 9 and 10, right). G1F decreased slightly when localized on FP1, but stayed constant when bound to FP2 (Figures 9 and 10, right). The same observation was made for G0F (Figures 9 and 10, right). These results clearly indicate that FP1 and FP2 with terminal galactosylated G2F were specifically removed from circulation. The mechanism behind this clearance could be the asialoglycoprotein receptor. This receptor binds selectively to exposed galactose residues. The receptor furthermore exhibits decreasing affinity from high to small number of terminal galactose residues, which could explain the observations for the less pronounced decrease or constant portion of G1F respectively.

The difference between FP1 and FP2 for the G1F and G0F N-glycan may occur due to different N-glycosylation site occupancy on the protein and a different accessibility for receptors to bind.

### Capping N-glycans with sialic acids at the receptor part increased half-life

As described earlier the major N-glycan SG2F had a slight trend towards higher relative percentages. At FP1 the amount of SG2 increased over time and at FP2 and SG1F showed the same trend. The other acidic N-glycan had constant percentages (Figures 9 and 10, right).

These observations lead to the conclusion that the addition of at least one terminal sialic acid to the galactosylated N-glycan structure may prevent the protein from selective clearance. The discrepancy between FP1 and FP2 with respect to the different N-glycans that increased over time may again occur due to differences in the glycosylation site where the structures are attached.

### Sialylation, galactosylation and terminal GlcNAc of FP1 and FP2

An overview over the terminal groups of all attached N-glycans is given in Figure 11. Minor abundant N-glycans were also included. The N-glycans were divided into three different groups: The group sialylation contains all N-glycans with one or two terminal sialic acids, the group galactosylation contains all N-glycans with at least one terminal galactose moiety and the group terminal GlcNAc contains N-glycans with a terminal N-acetylglucosamine. For N-glycans with two characteristics it had to be decided which attribute is more likely to have an influence on PK, e.g. for SG1F which has a terminal sialic acid on one arm and a terminal GlcNAc on the other arm it was decided that it is part of the sialylation group because it is more likely that this N-glycan behaves more like a sialic glycan than like a terminal GlcNAc glycan due to the shielding effect of the attached terminal sialic acid group. The same applies for G1F where the terminal galactose residue has a higher impact than the terminal GlcNAc.

Terminal groups did not change at the Fc parts of FP1 and FP2. The two deviations at 6 h (FP2) or at 32 h (FP1) were most probably due to method variation at low target protein concentrations. In contrast to the Fc part, the terminal groups at the receptor part showed decreasing galactosylation which was compensated with increasing terminal sialylation. N-glycans belonging to the GlcNAc group were constant.

### Conclusion

A work-flow has been established for the recovery of a fusion protein from serum samples. In a first affinity step using Protein G chromatography the complexity of the serum was drastically reduced. Serum related proteins that tend to bind non-specific to the sepharose resin were removed as well as all non IgG Fc part containing proteins. The pre-cleared serum was applied to antigen columns and after washing and enzymatic elution of the Fc part, the N-glycans of the two protein parts were analyzed individually. Due to the incorporation of stable heavy isotope 2-AA labeled glycan standards, variations during N-glycan processing and nanoLCMS analysis were compensated, and the time course of individual glycans in the study were determined with high precision.

Two different batches of a fusion protein were compared in a rabbit study and the PK profiles of each N-glycan and the relative distribution of the N-glycans over time were determined. The results show that fusion proteins with terminal galactosylated N-glycans that are located at the receptor part are cleared faster than the average molecule and that terminal sialic acids increase the serum half-life. This clearance is most likely a receptor mediated process of the asialoglycprotein receptor, which is located in the liver and known for its affinity to terminal galactosylated N-glycans.

In summary, this study showed that N-glycosylation of fusion proteins influences the PK. Terminal galactosylated N-glycans are cleared faster whereas terminal sialylation increases serum half-life. This is most likely due to asialo-glycoprotein receptor mediated binding and occurs only if the N-glycans are located at the receptor part. Fc part N-glycosylation (complex bi-antennary) has no influence on PK which is in agreement with previous studies. Thus, this method allows detailed site specific N-glycan PK profiling of biopharmaceuticals. It was further demonstrated that this kind of investigation is advantageous in contrast to N-glycan profiling without site or domain specificity.

## Claims

1. A method of analyzing glycans of a recombinant glycoprotein of interest in liquid samples of a mammal comprising
a) immobilizing the recombinant glycoprotein of interest comprised in each of two or more liquid samples on a separate solid support coupled to an affinity ligand specific for the recombinant glycoprotein in the samples;
b) releasing a glycan containing fragment of the recombinant glycoprotein of each of said samples from the solid support into separate eluates by enzymatic cleavage of the recombinant glycoprotein using an endoproteinase;
c) optionally releasing the glycans from the glycan containing fragment of the recombinant glycoprotein in the separate eluates;
d) labeling the glycans of each of said samples with a first stable isotope of a fluorescent label; and
e) analyzing the labeled glycans of step d) of each of said samples separately using LC-MS and comparing said two or more samples,
wherein a reference standard comprising glycans labeled with a second stable isotope of the fluorescent label is added to each of said samples prior to step b), c), d) or e) and the reference standard is analyzed together with the labeled glycans of step e).

2. The method of claim 1, wherein the recombinant glycoprotein is an Fc-fusion protein or an antibody and wherein the method further comprises analyzing the glycans of a second glycan containing fragment of the recombinant glycoprotein of each of said samples that remains immobilized to the solid support in step b), comprising the following steps:
aa) pre-clearing each of the two or more liquid samples of a mammal to remove glycoproteins binding non-specifically to the solid support prior to step a), comprising
aai) immobilizing Fc-containing glycoproteins on a solid support using an Fc-binding protein, wherein said Fc-binding protein is preferably selected from protein G or protein A, more preferably said Fc-binding protein is protein G; and
aaii) eluting the Fc-containing glycoproteins;
wherein the solid support used in said pre-clearing step is made of the same material as the solid support used in step a) of claim 1, but is not coupled to said affinity ligand.
cc) releasing the glycans from the immobilized second glycan containing fragment of the recombinant glycoprotein of each of said samples remaining on the solid support following step b) into separate solutions;
dd) labeling the glycans of each of said samples with a first stable isotope of a fluorescent label; and
ee) analyzing the labeled glycans of step dd) of each of said samples separately using LC-MS and comparing said two or more samples,
wherein a reference standard comprising glycans labeled with a second stable isotope of the fluorescent label is added to each of said samples prior to step cc), dd) or ee) and wherein the reference standard is analyzed together with the labeled glycans of step ee).

3. The method of any one of the preceding claims, wherein the fluorescent label is a ¹²[C] and ¹³[C] isotopic pair, preferably a ¹²[C₆] and ¹³[C₆] isotopic pair, more preferably a ¹²[C₆]-2-aminobenzoic acid (¹²[C₆]-2-AA) and a ¹³[C₆]-2-aminobenzoic acid (¹³[C₆]-2-AA) isotopic pair.

4. The method of any one of the preceding claims, wherein the recombinant glycoprotein is a fusion protein or an antibody, preferably an Fc-fusion protein or an antibody.

5. The method of claim 4, wherein the glycan containing fragment of the recombinant glycoprotein released from the solid support in step b) is an Fc domain.

6. The method of claim 4 or 5, wherein the recombinant glycoprotein is
(a) an antibody and the variable region of the antibody binds to the affinity ligand immobilized on the solid support, preferably the affinity ligand is an antigen; or
(b) an Fc-fusion protein comprising an Fc-domain and an effector domain and the effector domain binds to the affinity ligand immobilized on the solid support, wherein the affinity ligand is a binding partner or an antibody specifically binding to the effector domain of the Fc-fusion protein.

7. The method of any one of claims 4 to 6, wherein the enzyme used to release the glycan containing fragment of the recombinant glycoprotein from the solid support is an endoproteinase selected from the group consisting of papain, ficin, cysteine protease SpeB or cysteine proteinase IdeS.

8. The method of any one of the preceding claims, wherein
(a) the LC-MS is a reverse phase LC-MS or a NanoLC-MS, preferably the LC-MS is a reverse phase NanoLC-MS;
(b) the solid support is a resin comprising microbeads, preferably sepharose beads, agarose beads or magnetic beads, more preferably sepharose beads;
(c) the affinity ligand is coupled to the solid support via N-hydroxysuccinimide (NHS), cyanogen bromide, epoxy, carbodiimide or thiopropyl, preferably via N-hydroxysuccinimide (NHS); and/or
(d) the two or more liquid samples of a mammal are prepared for analysis in a multi well filter plate, preferably in a 24, 96 or 384 well filter plate, more preferably in a 96 well filter plate.

9. The method of any one of the preceding claims, wherein the liquid samples of a mammal
(a) are body fluids, preferably selected from the group consisting of serum or plasma, urine, cerebral spinal fluid, amniotic fluid, saliva, sweat, ejaculate, tears, phlegm, vaginal secretion, vaginal wash and colonic wash; preferably wherein said samples are plasma or serum samples; and/or
(b) are from a human, a monkey, a rodent, a dog, a cat or a pig.

10. The method anyone of the preceding claims, wherein pharmacokinetic parameters of at least one specific glycan structure of the glycans of the recombinant glycoprotein of interest are determined, preferably the Cₘₐₓ, tₘₐₓ, AUC or t_{1/2}.

11. The method of any one of the preceding claims, wherein the glycans are analyzed in an aliquot of each of said two or more liquid samples of a mammal and optionally the concentration of said recombinant glycoprotein is analyzed in a further aliquot of said two or more liquid samples of a mammal.

12. The method of any one of the preceding claims, wherein the samples or the aliquots to be analyzed have a volume from about 1 µl to about 1000 µl, from about 5 µl to about 500 µl, from about 10 µl to about 200 µl, from about 10 µl to about 100 µl, from about 25 µl to about 100 µl or from about 40 µl to about 75 µl, preferably of about 50 µl.

## Patentansprüche

1. Verfahren zum Analysieren von Glycanen eines rekombinanten Glycoproteins von Interesse in flüssigen Proben eines Säugetieres, umfassend
a) Immobilisieren des rekombinanten Glycoproteins von Interesse, das in jeder von zwei oder mehr flüssigen Proben enthalten ist, auf einem separaten festen Träger, der mit einem Affinitätsliganden gekoppelt ist, der spezifisch für das rekombinante Glycoprotein in den Proben ist;
b) Freisetzen eines glycanhaltigen Fragments des rekombinanten Glycoproteins von jeder der Proben von dem festen Träger in separate Eluate durch enzymatisches Spalten des rekombinanten Glycoproteins unter Verwendung einer Endoproteinase;
c) optionales Freisetzen der Glycane von dem glycanhaltigen Fragment des rekombinanten Glycoproteins in den separaten Eluaten;
d) Markieren der Glycane von jeder der Proben mit einem ersten stabilen Isotop einer fluoreszenten Markierung; und
e) separates Analysieren der markierten Glycane aus Schritt d) aus jeder der Proben unter Verwendung von LC-MS und Vergleichen der zwei oder mehr Proben,
wobei ein Referenzstandard, der Glycane umfasst, die mit einem zweiten stabilen Isotop der fluoreszenten Markierung markiert sind, vor dem Schritt b), c), d) oder e) jeder der Proben zugesetzt wird und der Referenzstandard zusammen mit den markierten Glycanen von Schritt e) analysiert wird.

2. Verfahren nach Anspruch 1, wobei das rekombinante Glycoprotein ein Fc-Fusionsprotein oder ein Antikörper ist und wobei das Verfahren ferner das Analysieren der Glycane eines zweiten glycanhaltigen Fragments des rekombinanten Glycoproteins von jeder der Proben umfasst, das in Schritt b) an dem festen Träger immobilisiert verbleibt, umfassend die folgenden Schritte:
aa) vor dem Schritt a) Vorklären von jeder der zwei oder mehr flüssigen Proben eines Säugetieres, um Glycoproteine zu entfernen, die nichtspezifisch an den festen Träger binden, umfassend
aai) Immobilisieren von Fc-haltigen Glycoproteinen auf einem festen Träger unter Verwendung eines Fc-bindenden Proteins, wobei das Fc-bindende Protein vorzugsweise aus Protein G oder Protein A ausgewählt wird, wobei das Fc-bindende Protein stärker bevorzugt Protein G ist; und
aaii) Eluieren der Fc-haltigen Glycoproteine;
wobei der feste Träger, der in dem Vorklärungsschritt verwendet wird, aus dem gleichen Material wie der feste Träger hergestellt ist, der in Schritt a) von Anspruch 1 verwendet wird, aber nicht an den Affinitätsliganden gekoppelt ist;
cc) Freisetzen der Glycane von dem immobilisierten zweiten glycanhaltigen Fragment des rekombinanten Glycoproteins von jeder der Proben, die nach Schritt b) auf dem festen Träger verbleiben, in separate Lösungen;
dd) Markieren der Glycane von jeder der Proben mit einem ersten stabilen Isotop einer fluoreszenten Markierung; und
ee) separates Analysieren der markierten Glycane von Schritt dd) von jeder der Proben unter Verwendung von LC-MS und Vergleichen der zwei oder mehr Proben,
wobei ein Referenzstandard, der Glycane umfasst, die mit einem zweiten stabilen Isotop der fluoreszenten Markierung markiert sind, vor dem Schritt cc), dd) oder ee) jeder der Proben zugesetzt wird und wobei der Referenzstandard zusammen mit den markierten Glycanen von Schritt ee) analysiert wird.

3. Verfahren nach einem der vorhergehenden Ansprüche, wobei die fluoreszente Markierung ein ¹²[C]- und ¹³[C]-Isotopenpaar, vorzugsweise ein ¹²[C₆]- und ¹³[C₆]-Isotopenpaar, stärker bevorzugt ein ¹²[C₆]-2-Aminobenzoesäure-C²[C₆]-2-AA)- und ein ¹³[C₆]-2-Aminobenzoesäure-(¹³[C₆]-2-AA)-Isotopenpaar ist.

4. Verfahren nach einem der vorhergehenden Ansprüche, wobei das rekombinante Glycoprotein ein Fusionsprotein oder ein Antikörper, vorzugsweise ein Fc-Fusionsprotein oder ein Antikörper, ist.

5. Verfahren nach Anspruch 4, wobei das glycanhaltige Fragment des rekombinanten Glycoproteins, das in Schritt b) von dem festen Träger freigesetzt wird, eine Fc-Domäne ist.

6. Verfahren nach Anspruch 4 oder 5, wobei das rekombinante Glycoprotein Folgendes ist:
(a) ein Antikörper, wobei die variable Region des Antikörpers an den Affinitätsliganden bindet, der auf dem festen Träger immobilisiert ist, wobei der Affinitätsligand vorzugsweise ein Antigen ist; oder
(b) ein Fc-Fusionsprotein, umfassend eine Fc-Domäne und eine Effektor-Domäne, wobei die Effektor-Domäne an den Affinitätsliganden bindet, der auf dem festen Träger immobilisiert ist, wobei der Affinitätsligand ein Bindungspartner oder ein Antikörper ist, der spezifisch an die Effektor-Domäne des Fc-Fusionsproteins bindet.

7. Verfahren nach einem der Ansprüche 4 bis 6, wobei das Enzym, das verwendet wird, um das glycanhaltige Fragment des rekombinanten Glycoproteins von dem festen Träger freizusetzen, eine Endoproteinase ist, die aus der Gruppe ausgewählt wird, die aus Papain, Ficin, Cysteinprotease SpeB oder Cysteinproteinase IdeS besteht.

8. Verfahren nach einem der vorhergehenden Ansprüche, wobei
(a) die LC-MS eine Umkehrphasen-LC-MS oder eine NanoLC-MS ist, wobei die LC-MS vorzugsweise eine Umkehrphasen-NanoLC-MS ist;
(b) der feste Träger ein Harz ist, das Mikrokügelchen umfasst, vorzugsweise Sepharose-Kügelchen, Agarose-Kügelchen oder magnetische Kügelchen, stärker bevorzugt Sepharose-Kügelchen;
(c) der Affinitätsligand über N-Hydroxysuccinimid (NHS), Cyanogenbromid, Epoxid, Carbodiimid oder Thiopropyl, vorzugsweise über N-Hydroxysuccinimid (NHS), an den festen Träger gekoppelt ist; und/oder
(d) die zwei oder mehr flüssigen Proben eines Säugetieres für die Analyse in einer Multiwell-Filterplatte, vorzugsweise in einer 24-, 96- oder 384- Well-Filterplatte, stärker bevorzugt in einer 96-Well- Filterplatte, vorbereitet werden.

9. Verfahren nach einem der vorhergehenden Ansprüche, wobei die flüssigen Proben eines Säugetieres
(a) Körperflüssigkeiten sind, die vorzugsweise aus der Gruppe ausgewählt werden, die aus Serum oder Plasma, Harn, Zerebrospinalflüssigkeit, Fruchtwasser, Speichel, Schweiß, Ejakulat, Tränen, Schleim, Vaginalsekret, Vaginalspülung und Kolonspülung besteht; wobei die Proben vorzugsweise Plasma- oder Serumproben sind; und/oder
(b) von einem Menschen, einem Affen, einem Nagetier, einem Hund, einer Katze oder einem Schwein stammen.

10. Verfahren nach einem der vorhergehenden Ansprüche, wobei pharmakokinetische Parameter von wenigstens einer spezifischen Glycanstruktur der Glycane des rekombinanten Glycoproteins von Interesse bestimmt werden, vorzugsweise die Cₘₐₓ, tₘₐₓ, AUC oder t_{1/2}.

11. Verfahren nach einem der vorhergehenden Ansprüche, wobei die Glycane in einem Aliquot von jeder der zwei oder mehr flüssigen Proben eines Säugetieres analysiert werden und optional die Konzentration des rekombinanten Glycoproteins in einem weiteren Aliquot der zwei oder mehr flüssigen Proben eines Säugetieres analysiert wird.

12. Verfahren nach einem der vorhergehenden Ansprüche, wobei die zu analysierenden Proben oder Aliquote ein Volumen von etwa 1 µl bis etwa 1000 µl von etwa 5 µl bis etwa 500 µl von etwa 10 µl bis etwa 200 µl von etwa 10 µl bis etwa 100 µl von etwa 25 µl bis etwa 100 µl oder von etwa 40 µl bis etwa 75 µl, vorzugsweise von etwa 50 µl, aufweisen.

## Revendications

1. Procédé d'analyse de glycanes d'une glycoprotéine recombinante d'intérêt dans des échantillons liquides d'un mammifère comprenant :
a) l'immobilisation de la glycoprotéine recombinante d'intérêt comprise dans chacun de deux échantillons liquides ou plus sur un support solide séparé couplé à un ligand d'affinité spécifique de la glycoprotéine recombinante dans les échantillons ;
b) la libération d'un fragment contenant des glycanes de la glycoprotéine recombinante de chacun desdits échantillons du support solide dans des éluats séparés par clivage enzymatique de la glycoprotéine recombinante en utilisant une endoprotéinase ;
c) facultativement la libération des glycanes du fragment contenant des glycanes de la glycoprotéine recombinante dans les éluats séparés ;
d) le marquage des glycanes de chacun desdits échantillons avec un premier isotope stable d'un marqueur fluorescent ; et
e) l'analyse des glycanes marqués de l'étape d) de chacun desdits échantillons séparément en utilisant la CL-SM et en comparant lesdits deux échantillons ou plus,
dans lequel un étalon de référence comprenant les glycanes marqués avec un second isotope stable du marqueur fluorescent est ajouté à chacun desdits échantillons avant l'étape b), c), d) ou e) et l'étalon de référence est analysé conjointement aux glycanes marqués de l'étape e).

2. Procédé selon la revendication 1, dans lequel la glycoprotéine recombinante est une protéine de fusion à Fc ou un anticorps et dans lequel le procédé comprend en outre l'analyse des glycanes d'un second fragment contenant des glycanes de la glycoprotéine recombinante de chacun desdits échantillons qui reste immobilisé sur le support solide à l'étape b), comprenant les étapes suivantes :
aa) le pré-nettoyage de chacun des deux échantillons liquides ou plus d'un mammifère pour éliminer les glycoprotéines ne se liant pas spécifiquement au support solide avant l'étape a), comprenant :
aai) l'immobilisation de glycoprotéines contenant Fc sur un support solide en utilisant une protéine de liaison à Fc, dans lequel ladite protéine de liaison à Fc est de préférence sélectionnée parmi la protéine G ou la protéine A, de manière davantage préférée la protéine de liaison à Fc est la protéine G ; et
aaii) l'élution des glycoprotéines contenant Fc ;
dans lequel le support solide utilisé dans ladite étape de pré-nettoyage est constitué du même matériau que le support solide utilisé à l'étape a) de la revendication 1, mais n'est pas couplé au dit ligand d'affinité ;
cc) la libération des glycanes du second fragment contenant des glycanes immobilisé de la glycoprotéine recombinante de chacun desdits échantillons restant sur le support solide après l'étape b) dans des solutions séparées ;
dd) le marquage des glycanes de chacun desdits échantillons avec un premier isotope stable d'un marqueur fluorescent ; et
ee) l'analyse des glycanes marqués de l'étape dd) de chacun desdits échantillons séparément en utilisant la CL-SM et en comparant lesdits deux échantillons ou plus,
dans lequel un étalon de référence comprenant des glycanes marqués avec un second isotope stable du marqueur fluorescent est ajouté à chacun desdits échantillons avant les étapes cc), dd) ou ee) et dans lequel l'étalon de référence est analysé conjointement aux glycanes marqués de l'étape ee).

3. Procédé selon l'une quelconque des revendications précédentes, dans lequel le marqueur fluorescent est une paire isotopique ¹²[C] et ¹³[C], de préférence une paire isotopique ¹²[C₆] et ¹³[C₆], de manière davantage préférée une paire isotopique ¹²[C₆]-acide 2-aminobenzoïque (¹²[C₆]-2-AA) et ¹³[C₆]-acide 2-aminobenzoïque (¹³[C₆]-2-AA).

4. Procédé selon l'une quelconque des revendications précédentes, dans lequel la glycoprotéine recombinante est une protéine de fusion ou un anticorps, de préférence une protéine de fusion à Fc ou un anticorps.

5. Procédé selon la revendication 4, dans lequel le fragment contenant des glycanes de la glycoprotéine recombinante libérée du support solide à l'étape b) est un domaine Fc.

6. Procédé selon la revendication 4 ou 5, dans lequel la glycoprotéine recombinante est
(a) un anticorps et la région variable de l'anticorps se lie au ligand d'affinité immobilisé sur le support solide, de préférence le ligand d'affinité est un antigène ; ou
(b) une protéine de fusion à Fc comprenant un domaine Fc et un domaine effecteur et le domaine effecteur se lie au ligand d'affinité immobilisé sur le support solide, dans lequel le ligand d'affinité est un partenaire de liaison ou un anticorps se liant spécifiquement au domaine effecteur de la protéine de fusion à Fc.

7. Procédé selon l'une quelconque des revendications 4 à 6, dans lequel l'enzyme utilisée pour libérer le fragment contenant des glycanes de la glycoprotéine recombinante du support solide est une endoprotéinase sélectionnée dans le groupe constitué de la papaïne, de la ficine, de la cystéine protéase SpeB ou de la cystéine protéinase IdeS.

8. Procédé selon l'une quelconque des revendications précédentes, dans lequel
(a) la CL-SM est une CL en phase inverse-SM, ou une nano-CL-SM, de préférence, la CL-SM est une nano-CL en phase inverse-SM ;
(b) le support solide est une résine comprenant des microbilles, de préférence des billes de sépharose, des billes d'agarose ou des billes magnétiques, de manière davantage préférée des billes de sépharose ;
(c) le ligand d'affinité est couplé au support solide via le N-hydroxysuccinimide (NHS), le bromure de cyanogène, un époxy, un carbodiimide ou un thiopropyle, de préférence via le N-hydroxysuccinimide (NHS) ; et/ou
(d) les deux échantillons liquides ou plus d'un mammifère sont préparés pour analyse dans une plaque de filtration à puits multiples, de préférence dans une plaque de filtration à 24, 96 ou 384 puits, de manière davantage préférée dans une plaque de filtration à 96 puits.

9. Procédé selon l'une quelconque des revendications précédentes, dans lequel les échantillons liquides d'un mammifère
(a) sont des fluides corporels, de préférence sélectionnés dans le groupe constitué de sérum ou de plasma, d'urine, de liquide céphalo-rachidien, de liquide amniotique, de salive, de transpiration, d'éjaculat, de larmes, de phlegme, de sécrétions vaginales, de lavage vaginal et de lavage du côlon ; de préférence dans lequel lesdits échantillons sont des échantillons de plasma ou de sérum ; et/ou
(b) proviennent d'un être humain, d'un singe, d'un rongeur, d'un chien, d'un chat ou d'un porc.

10. Procédé selon l'une quelconque des revendications précédentes, dans lequel les paramètres pharmacocinétiques d'au moins une structure de glycane spécifique des glycanes de la glycoprotéine recombinante d'intérêt sont déterminés, de préférence les Cₘₐₓ, tₘₐₓ, ASC ou t_{1/2}.

11. Procédé selon l'une quelconque des revendications précédentes, dans lequel les glycanes sont analysés dans une aliquote de chacun desdits deux échantillons liquides ou plus d'un mammifère et facultativement la concentration de ladite glycoprotéine recombinante est analysée dans une autre aliquote desdits deux échantillons liquides ou plus d'un mammifère.

12. Procédé selon l'une quelconque des revendications précédentes, dans lequel les échantillons ou les aliquotes à analyser ont un volume d'environ 1 µl à environ 1 000 µl, d'environ 5 µl à environ 500 µl, d'environ 10 µl à environ 200 µl, d'environ 10 µl à environ 100 µl, d'environ 25 µl à environ 100 µl, ou d'environ 40 µl à environ 75 µl, de préférence d'environ 50 µl.
